# EUROPEAN PATENT APPLICATION

(11) **EP 4 306 646 A1**
(43) Date of publication of application: **17.01.2024**
(21) Application number: 22184055.6
(22) Date of filing: 11.07.2022
(51) Int. Cl.: C12P 19/30, C12P 19/38

(54) **METHOD AND MEANS FOR SYNTHESIS OF C-NUCLEOSIDES OR C-NUCLEOTIDES**

(71) Applicant: Technische Universität Graz, 8010 Graz (AT)
(72) Inventor: NIDETZKY, Bernd, 8010 Graz (AT); PFEIFFER, Martin, 8010 Graz (AT)
(74) Representative: Gassner, Birgitta

(57) **Abstract**

The invention refers to method and means for the production of C-nucleotides or C-nucleoside from the corresponding N-nucleoside. Specifically provided is a method for the production of a C-nucleotide or C-nucleoside from the corresponding N-nucleoside, said method comprising the steps of:
a) providing an N-nucleoside and a phosphate donor;
b) adding an enzyme having phosphorylase activity, whereupon the N-nucleoside is converted into the corresponding nucleobase and sugar-phosphate moiety;
c) adding an enzyme having isomerase activity, whereupon the sugar-phosphate moiety is subjected to an intramolecular phosphoryl transfer; and
d) adding an enzyme having C-glycosidase activity, whereupon the nucleobase and the rearranged sugar-phosphate moiety yield the corresponding C-nucleoside-monophosphate.

## Description

### Field of the Invention

The present invention relates to the field of biocatalysis, in particular to the enzymatic synthesis of nucleoside or nucleotide analogs, specifically C-nucleosides and C-nucleotides. The present invention relates to methods and means for catalyzing the biocatalytic synthesis of C-nucleosides or C-nucleotides from their corresponding N-nucleosides.

### Background Art

C-nucleotides are C-analogs of canonical N-nucleotides and have considerable importance in medicinal chemistry. Compared to N-nucleotides, C-nucleotides resist biological degradation by N-glycoside hydrolases and phosphorylases.

C-nucleoside analogs are promising lead structures in drug design for a variety of applications such as for antiviral, antibacterial, and antitumor activity but also as building blocks of nucleic acids, in particular as building blocks for RNA.

Since the breakthrough of mRNA vaccination technology, pharmaceutical mRNA preparations are becoming increasingly important. In these pharmaceutical mRNA preparations, the canonical uridine is replaced by the C-nucleoside 1-N-methyl-pseudouridine to reduce the immunogenicity and ensure optimal translation. The uniform substitution of uridine (U) by pseudouridine (Ψ) can render synthetic mRNA non-immunogenic and was shown to enhance the translation efficiency.

The positional substitution of U with Ψ increases RNA stability by locally enhancing the macromolecular rigidity. In pseudouridine, additional hydrogen bonding is formed from the nucleobase N1 in Ψ compared to U.

Additionally, it has been shown that Ψ-modified mRNA can boost the gene-cutting efficiency in CRISPR-based gene editing.

Pseudouridine (Ψ) is the most abundant modification in RNA and exists in tRNAs, rRNAs, snRNAs, snoRNAs, scaRNAs, and mRNA. Pseudouridine is synthesized *in situ* by pseudouridine synthase (Ψ-synthase) which post-translationally isomerizes U to Ψ. Thereby, the synthesis of pseudouridine is catalyzed by pseudouridine synthases which catalyzes the site-specific isomerization of uridine residues, wherein the uridine residue is part of an RNA chain. Thus, pseudouridine synthases catalyzes the isomerization of uridine to pseudouridine in an RNA chain. The isomerization of a free uridine molecule to pseudouridine is not catalyzed by pseudouridine synthase.

Pfeiffer, M. & Nidetzky, B. (2020) described the synthesis of pseudouridine monophosphate from unprotected sugar and uracil as substrates using several different kinases and a Ψ MP-glycosidase [1]. Incorporation of the synthesized C-nucleotide triphosphates into nucleic acids by RNA polymerase has also been described. However, this process requires the use of kinases and requires starting materials which are not easily available. Thereby, an expensive phosphate donor including its regeneration system is needed leading to high production costs for pseudouridine.

Chemical synthesis of C-nucleotides either follows reconstituting the heteroaryl nucleobase on a C1'-functionalized sugar or couple the prepared heteroaryl with a suitable glycosyl reagent. These approaches involve multistep reactions with low overall yield and limited stereoselectivity resulting in the production of unwanted side products such as alpha-D-pseudouridine.

However, methods and means for synthesizing these C-nucleosides and/or C-nucleotides are currently lacking. The demand for these C-nucleosides such as pseudouridine is steadily increasing.

Thus, there is an unmet need for an efficient and structurally precise synthetic method of C-nucleoside production. There is also a need for such a synthesis method which is based on readily available starting materials.

### Summary of the invention

It is the object of the present invention to provide an efficient and stereoselective production method for C-nucleosides and/or C-nucleotides from easily available substrates.

The object is solved by the subject matter of the present invention.

According to the invention there is provided a method for the production of a C-nucleotide or C-nucleoside from the corresponding N-nucleoside, said method comprising the steps of:
a) providing an N-nucleoside and a phosphate donor;
b) adding an enzyme having phosphorylase activity, whereupon the N-nucleoside is converted into the corresponding nucleobase and sugar-phosphate moiety;
c) adding an enzyme having isomerase activity, whereupon the sugar-phosphate moiety is subjected to an intramolecular phosphoryl transfer; and
d) adding an enzyme having C-glycosidase activity, whereupon the nucleobase and the rearranged sugar-phosphate moiety yield the corresponding C-nucleoside-monophosphate.

According to one embodiment, the method is performed as a one-pot reaction.

According to a further embodiment, the N-nucleoside is a pyrimidine nucleoside or a derivative thereof, preferably uridine, cytidine, or a derivative thereof.

The uridine derivative is of general formula I, 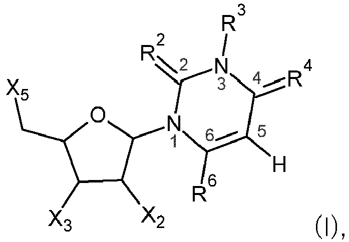 wherein
R²denotes O or S;
R³ denotes H, CH₃ or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
R⁴ denotes O or S;
R⁶denotes H, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X₂ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl; and
X₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl.

The cytidine derivative is of general formula II 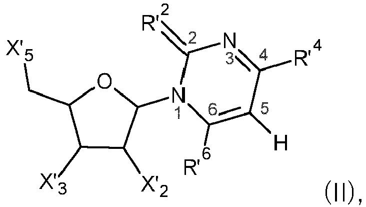 wherein
R'² denotes O or S;
R'⁴ denotes NH₂, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
R'⁶denotes H, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X'₂denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X'₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl; and
X'₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl.

The phosphate donor may be an inorganic phosphate, preferably phosphoric acid or a salt thereof, more preferably H₃PO₄, H₂PO₄ , HPO₄²⁻ or PO₄³⁻ .

The enzyme having phosphorylase activity may be a uridine phosphorylase or pyrimidine-nucleoside phosphorylase; for example EcUP, EcPNP, or a functionally active variant thereof.

The enzyme having isomerase activity may be a phospho-mutase; for example a phosphopentose-mutase; such as DeoB, or a functionally active variant thereof.

The enzyme having C-glycosidase activity may be pseudouridine-monophosphate(Ψ MP)-glycosidase; such as YeiN or a functionally active variant thereof.

According to one embodiment of the invention, the method comprises the following steps of:
a) providing uridine and a phosphate donor;
b) adding an enzyme having uridine phosphorylase activity, whereupon uridine is converted into uracil and ribose 1-phosphate;
c) adding an enzyme having phosphopentose-mutase activity, whereupon ribose 1-phosphate is converted to ribose 5-phosphate; and
d) adding an enzyme having Ψ MP-glycosidase activity, whereupon uracil and ribose 5-phosphate yield pseudouridine monophosphate (Ψ MP);
preferably said method is performed as a one-pot reaction.

The method described herein optionally further comprises the step of adding an enzyme having phosphatase activity, whereupon the phosphate group is removed from the C-nucleotide yielding the corresponding C-nucleoside, preferably pseudouridine (Ψ).

The enzyme having phosphatase activity may be calf intestine phosphatase (CIP), pyrimidine nucleoside monophosphate phosphatase (Yjjg), or a functionally active variant thereof.

According to one embodiment of the invention, the N1 atom of the nucleobase in the C-nucleotide or in the C-nucleoside is substituted by a suitable moiety.

According to the invention there is further provided a use of an enzyme mixture comprising an enzyme having phosphorylase activity, an enzyme having isomerase activity, an enzyme having C-glycosidase activity, and optionally an enzyme having phosphatase activity for the production of a C-nucleotide or a C-nucleoside. In one embodiment the enzyme having phosphatase activity is Yjjg.

According to the invention there is further provided a cell adapted to co-express the enzyme mixture described herein.

### Brief description of drawings

Figure 1: Time course of Ψ MP synthesis
Figure 2: a) Ψ MP after lyophilization, b) Product identification: HPLC chromatogram of the Ψ MP cascade reaction after 30 hours of incubation.
Figure 3: Time course of Ψ synthesis
Figure 4: a) produced Ψ, b) Ψ after lyophilization, c) HPLC chromatogram of the Ψ cascade reaction after 22 hours of incubation.
Figure 5: Time course of Ψ TP synthesis
Figure 6: HPLC analysis of Ψ TP

### Description of embodiments

The present invention provides methods and means for the production of C-nucleosides or C-nucleotides from easily available substrates using an efficient biocatalytic cascade reaction. Thereby, enzymes are used for catalyzing distinct reactions enabling the production of a single isomeric product without the need of using protection groups. Specifically, methods and means for pseudouridine-monophosphate and pseudouridine production are provided herein starting from uridine as substrate in a chemically efficient transformation reaction which eliminates the major hurdle of substrate supply. Thereby, an enzymatic cascade reaction is provided herein to achieve the conversion of a N-nucleoside to a C-nucleoside or C-nucleotide such as the conversion of uridine to pseudouridine-monophosphate or to pseudouridine.

The inventors of the present invention surprisingly found that the transformation of uridine to pseudouridine or pseudouridine-monophosphate using the methods and means of the invention occurred in an unusual and unexpected efficiency. Surprisingly, using the method described herein, uridine can be converted completely with a 100% yield into pseudouridine. Furthermore, no byproducts are formed in the method described herein.

Further, the authors of the present invention surprisingly found that in the method provided herein the nucleobase can be cleaved from uridine by an enzyme having phosphorylase activity, thereby generating a ribose 1-phosphate. Subsequently, an enzyme having phosphopentose mutase is converting the ribose 1-phosphate to ribose 5-phosphate. And finally, an enzyme having Ψ MP-glycosidase activity is reconnecting the nucleobase to the ribose through C5. Moreover, these catalytic steps can be performed in a one-pot reaction.

Thereby, the present invention provides the advantageous effects that expensive substrates, multiple steps of processing, more complex reactions, and protection-deprotection chemistry are avoided. The method for the production of C-nucleosides provided herein is characterized by a high yield, high efficiency, and eco-efficiency.

The subject matter of the claims refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, expression constructs, transformed host cells and modified proteins and enzymes, are "man-made" or synthetic, and are therefore not considered as a result of "laws of nature".

According to one embodiment of the invention, a C-nucleotide or C-nucleoside is produced from the corresponding N-nucleoside e.g., the C-nucleoside pseudouridine is produced from the corresponding N-nucleoside uridine.

According to one embodiment of the invention, the method described herein provides the advantage of stereo-control giving only beta-D-pseudouridine as single product. Furthermore, there is no need for expensive phosphate-donors. Also, the method described herein provides an excellent overall yield of >95% over all steps of the method. A high product titer of >50 g/L is reached using the method provided herein. Also, no toxic chemicals are needed using the method described herein.

Uridine is an N-nucleoside of the following chemical structure (the numbering of the atoms in the nucleobase as used herein is shown):

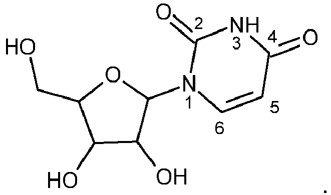

Pseudouridine is the corresponding C-nucleoside of the N-nucleoside uridine in which the uracil is attached via a carbon-carbon instead of a nitrogen-carbon glycosidic bond. Pseudouridine is also referred to as 5-ribosyluracil. Pseudouridine is abbreviated herein as "Ψ". Pseudouridine has the following chemical structure (the numbering of the atoms in the nucleobase as used herein is shown):

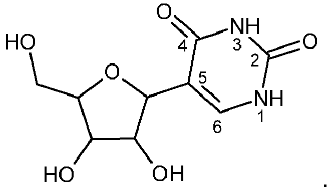

Pseudouridine monophosphate is an example of a corresponding C-nucleotide of the N-nucleoside uridine. Pseudouridine monophosphate is abbreviated herein as " Ψ MP" and refers to pseudouridine comprising one phosphate group attached to the sugar. Pseudouridine monophosphate has the following chemical structure:

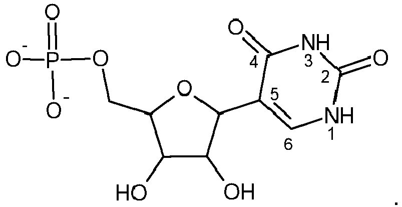

According to the invention, a C-nucleoside or C-nucleotide is produced from the corresponding N-nucleoside through enzyme-catalyzed reactions.

According to one embodiment of the invention, an enzyme having phosphorylase activity is added in the method described herein. An enzyme having phosphorylase activity catalyzes the addition of a phosphate group to an acceptor. As used herein, the enzyme having phosphorylase activity catalyzes the addition of phosphate to the sugar of an N-nucleoside, whereupon the N-nucleoside is converted into the corresponding nucleobase and the sugar-phosphate moiety. Thus, any enzyme able to catalyze the addition of a phosphate group to the sugar of a N-nucleoside may be used according to the invention. In other words, any enzyme able to catalyze the following reaction may be used according to the invention:

N-nucleoside + phosphate ⇄ nucleobase + sugar 1-phosphate.

Non-limiting examples of enzymes having phosphorylase activity are uridine phosphorylases (UP) and purine/pyrimidine-nucleoside phosphorylases (PNP).

Enzymes having pyrimidine-nucleoside phosphorylase (PNP) activity catalyze the following reactions:

uridine + phosphate ⇄ uracil + alpha-D-ribose 1-phosphate;

cytidine + phosphate ⇄ cytosine + alpha-D-ribose 1-phosphate;

2'-deoxyuridine + phosphate ⇄ uracil + 2-deoxy-alpha-D-ribose 1-phosphate; and/or

thymidine + phosphate ⇄ thymine + 2-deoxy-alpha-D-ribose 1-phosphate.

According to another specific embodiment, enzymes having uridine phosphorylase (UP) activity catalyze the following reaction

uridine + phosphate ⇄ uracil + alpha-D-ribose 1-phosphate.

Enzymes having pyrimidine-nucleoside phosphorylase (PNP) activity are for example derived from Escherichia coli (EcPNP).

An enzyme having uridine phosphorylase activity is uridine phosphorylase from *Escherichia coli* (SEQ ID NO: **1**, herein also referred to as EcUP).

According to one embodiment, an enzyme having phosphorylase activity is added to the reaction mixture, wherein said enzyme is uridine phosphorylase from Escherichia coli (EcUP) given in SEQ ID NO: **1**, or an enzyme with at least 5, 10, 20, 30, 40, 45, 50, 55, 60, 65, 70, 75, 80, 85, 90, 95, 98, 99, 100 %, or an even higher specific activity of the uridine phosphorylase activity of said EcUP. Alternatively, the enzyme having phosphorylase activity is a functionally active variant of SEQ ID NO: **1** having at least 10, 20, 30, 40, 45, 50, 55, 60, 65, 70, 75, 80, 85, 90, 95, 98, 99, or 100 % sequence identity with SEQ ID NO: **1**.

According to one embodiment, an enzyme having phosphorylase activity is added to the reaction mixture, wherein said enzyme is pyrimidine/purine nucleoside phosphorylase from Escherichia coli (EcPNP) given in SEQ ID NO: **2**, or an enzyme with at least 5, 10, 20, 30, 40, 45, 50, 55, 60, 65, 70, 75, 80, 85, 90, 95, 98, 99, 100 %, or an even higher specific activity of the pyrimidine/purine nucleoside phosphorylase activity of said EcPNP. Alternatively, the enzyme having phosphorylase activity is a functionally active variant of SEQ ID NO: **2** having at least 10, 20, 30, 40, 45, 50, 55, 60, 65, 70, 75, 80, 85, 90, 95, 98, 99, or 100 % sequence identity with SEQ ID NO: **2**.

According to one embodiment of the invention, an enzyme having isomerase activity is added to the reaction mixture. An enzyme having "isomerase activity" is an enzyme able to catalyze the conversion of a molecule from one isomer to another. An isomerase activity is characterized in that there is only one substrate yielding one product, wherein the product has the same molecular formula as the substrate but differs in bond connectivity or spatial arrangement.

The enzyme having isomerase activity may be an enzyme having phosphomutase or phosphotransferase activity which catalyzes the transfer of phosphate groups within a molecule. An enzyme having phosphomutase activity is for example a "phosphopentose-mutase". The systematic name of the enzyme class of phosphopentose-mutases is alpha-D-ribose 1,5-phosphomutase. Other names in common use include DeoB, phosphodeoxyribomutase, deoxyribose phosphomutase, deoxyribomutase, phosphoribomutase, alpha-D-glucose-1,6-bisphosphate:deoxy-D-ribose-1-phosphate, phosphotransferase, and D-ribose 1,5-phosphomutase. A phosphopentose-mutase catalyzes the following chemical reaction:

alpha-D-ribose 1-phosphate ⇄ D-ribose 5-phosphate.

Thereby, the substrate alpha-D-ribose 1-phosphate is converted into the product D-ribose 5-phosphate.

According to one embodiment, the enzyme having phosphopentose-mutase activity is DeoB (SEQ ID NO: **3**), or an enzyme with at least 5, 10, 20, 30, 40, 45, 50, 55, 60, 65, 70, 75, 80, 85, 90, 95, 98, 99, 100 %, or an even higher specific activity of the phosphopentose-mutase activity of SEQ ID NO: **3**. Alternatively, the enzyme having phosphopentose-mutase activity is a functionally active variant of SEQ ID NO: **3** having at least 10, 20, 30, 40, 45, 50, 55, 60, 65, 70, 75, 80, 85, 90, 95, 98, 99, or 100 % sequence identity with SEQ ID NO: **3**.

According to one embodiment of the invention, an enzyme having C-glycosidase activity is added to the reaction mixture. In general, glycosidases are also called glycoside hydrolases or glycosyl hydrolases and catalyze the hydrolysis of glycosidic bonds. In nature, glycosidases are involved in biomass degradation e.g., cellulase in cellulose degradation, and amylase in starch degradation. In the reverse reaction, glycosidases catalyze the formation of glycosidic bonds through reverse hydrolysis where the equilibrium position is reversed, or by transglycosylation whereby retaining glycoside hydrolases can catalyze the transfer of a glycosyl moiety from an activated glycoside to an acceptor alcohol to afford a new glycoside. In particular, the enzyme having C-glycosidase activity is able to catalyze the reverse reaction i.e., the catalysis of the reaction of nucleobase and sugar phosphate moiety yielding a C-nucleoside monophosphate.

According to one embodiment of the invention, a Ψ MP-glycosidase is used for the production of Ψ MP from ribose 5-phosphate and uracil. Thereby, the Ψ MP-glycosidase catalyzes the stereo-selective 5-*β* -C-glycosylation of uracil.

The reverse reaction of the Ψ MP-glycosidase for the synthesis of Ψ 5'-phosphate (Ψ MP) from D-ribose 5-phosphate and uracil is based on the following key catalytic steps: formation of a covalent iminium ion intermediate between the enzyme (Lys166) and the open-chain D-ribose 5-phosphate, assisted by Glu31 (I); Mannich-like reaction for C-C coupling (II); *β* -elimination of the amine of Lys166 (III); cyclization through an oxa-Michael addition-like reaction (IV) [1].

An enzyme having C-glycosidase activity may be an enzyme having pseudouridine monophosphate glycosidase activity catalyzing the reverse reaction of the cleavage of a C-C glycosidic bond yielding into pseudouridine monophosphate from uracil and ribose-5-phosphate.

According to one embodiment, the enzyme having C-glycosidase activity is YeiN (SEQ ID NO: **4**) or an enzyme with at least 5, 10, 20, 30, 40, 45, 50, 55, 60, 65, 70, 75, 80, 85, 90, 95, 98, 99, 100 %, or an even higher specific activity of the reverse C-glycosidase activity of SEQ ID NO: **4**. Alternatively, the enzyme having C-glycosidase activity is a functionally active variant of SEQ ID NO: **4** having at least 10, 20, 30, 40, 45, 50, 55, 60, 65, 70, 75, 80, 85, 90, 95, 98, 99, or 100 % sequence identity with SEQ ID NO: **4**.

According to one embodiment, the enzyme having C-glycosidase activity is IndA (SEQ ID NO: **13**) or an enzyme with at least 5, 10, 20, 30, 40, 45, 50, 55, 60, 65, 70, 75, 80, 85, 90, 95, 98, 99, 100 %, or an even higher specific activity of the reverse C-glycosidase activity of SEQ ID NO: **13**. Alternatively, the enzyme having C-glycosidase activity is a functionally active variant of SEQ ID NO: **13** having at least 10, 20, 30, 40, 45, 50, 55, 60, 65, 70, 75, 80, 85, 90, 95, 98, 99, or 100 % sequence identity with SEQ ID NO: **13**.

According to one embodiment, the enzyme having C-glycosidase activity is OzmD (SEQ ID NO: **14**) or an enzyme with at least 5, 10, 20, 30, 40, 45, 50, 55, 60, 65, 70, 75, 80, 85, 90, 95, 98, 99, 100 %, or an even higher specific activity of the reverse C-glycosidase activity of SEQ ID NO: **14.** Alternatively, the enzyme having C-glycosidase activity is a functionally active variant of SEQ ID NO: **14** having at least 10, 20, 30, 40, 45, 50, 55, 60, 65, 70, 75, 80, 85, 90, 95, 98, 99, or 100 % sequence identity with SEQ ID NO: **14.**

According to one embodiment, the enzyme having C-glycosidase activity is SdmA (SEQ ID NO: **15**) or an enzyme with at least 5, 10, 20, 30, 40, 45, 50, 55, 60, 65, 70, 75, 80, 85, 90, 95, 98, 99, 100 %, or an even higher specific activity of the reverse C-glycosidase activity of SEQ ID NO: **15.** Alternatively, the enzyme having C-glycosidase activity is a functionally active variant of SEQ ID NO: **15** having at least 10, 20, 30, 40, 45, 50, 55, 60, 65, 70, 75, 80, 85, 90, 95, 98, 99, or 100 % sequence identity with SEQ ID NO: **15.**

According to one embodiment, the enzyme having C-glycosidase activity is MinB (SEQ ID NO: **16**) or an enzyme with at least 5, 10, 20, 30, 40, 45, 50, 55, 60, 65, 70, 75, 80, 85, 90, 95, 98, 99, 100 %, or an even higher specific activity of the reverse C-glycosidase activity of SEQ ID NO: **16.** Alternatively, the enzyme having C-glycosidase activity is a functionally active variant of SEQ ID NO: **16** having at least 10, 20, 30, 40, 45, 50, 55, 60, 65, 70, 75, 80, 85, 90, 95, 98, 99, or 100 % sequence identity with SEQ ID NO: **16.**

For producing a C-nucleoside, the method further comprises adding an enzyme having phosphatase activity. This enzyme having phosphatase activity catalyzes the removal of the phosphate group from the C-nucleotide yielding the corresponding C-nucleoside.

In general, an enzyme having phosphatase activity catalyzes the hydrolysis of a phosphomonoester, removing a phosphate moiety from the substrate resulting in the destruction of a phosphomonoester and the creation of both a phosphate ion and a molecule with a free hydroxyl group.

According to one embodiment, an enzyme having phosphatase activity is pyrimidine nucleoside monophosphate phosphatase (Yjjg, SEQ ID NO: **5**), or an enzyme with at least 5, 10, 20, 30, 40, 45, 50, 55, 60, 65, 70, 75, 80, 85, 90, 95, 98, 99, 100 %, or an even higher specific activity of the phosphatase activity of SEQ ID NO: **5**. Alternatively, the enzyme having phosphatase activity is a functionally active variant of SEQ ID NO: **5** having at least 10, 20, 30, 40, 45, 50, 55, 60, 65, 70, 75, 80, 85, 90, 95, 98, 99, or 100 % sequence identity with SEQ ID NO: **5**.

According to one embodiment, the enzyme having phosphatase activity is calf intestine phosphatase (CIP, SEQ ID NO: **12**) or an enzyme with at least 5, 10, 20, 30, 40, 45, 50, 55, 60, 65, 70, 75, 80, 85, 90, 95, 98, 99, 100 %, or an even higher specific activity of the phosphatase activity of SEQ ID NO: **12**. Alternatively, the enzyme having phosphatase activity is a functionally active variant of SEQ ID NO: **12** having at least 10, 20, 30, 40, 45, 50, 55, 60, 65, 70, 75, 80, 85, 90, 95, 98, 99, or 100 % sequence identity with SEQ ID NO: **12.**

According to another embodiment, in the method described herein any enzyme having phosphatase activity may be used. Non-limiting examples of subfamilies of enzymes having phosphatase activity are histidine acid phosphatases, alkaline phosphatases, and haloacid dehalogenase-like phosphatase.

For the production of a C-nucleoside-diphosphate or a C-nucleoside-triphosphate , the method further comprises adding an enzyme having kinase activity, whereupon the transfer of one or more phosphate groups to the C-nucleoside or C-nucleotide is catalyzed.

According to one embodiment, pseudouridine-diphosphate (Ψ DP) or pseudouridine-triphosphate (Ψ TP) is produced according to the method described herein. Thereby, the production of Ψ MP from U using the three enzymes cascade comprising e.g., UP, DeoB, YeiN, is combined with the addition of ATP and an enzyme having nucleoside monophosphate kinase activity catalyzing the following reaction:

Ψ MP + ATP -> Ψ DP+ ADP.

For the production of Ψ DP, an enzyme having nucleoside monophosphate kinase activity is needed which may be cytidylate kinase given in SEQ ID NO: **6** or an enzyme with at least 5, 10, 20, 30, 40, 45, 50, 55, 60, 65, 70, 75, 80, 85, 90, 95, 98, 99, 100 %, or an even higher specific activity of the nucleoside monophosphate kinase activity of SEQ ID NO: **6.** Alternatively, the enzyme having nucleoside monophosphate kinase activity is a functionally active variant of SEQ ID NO: **6** having at least 10, 20, 30, 40, 45, 50, 55, 60, 65, 70, 75, 80, 85, 90, 95, 98, 99, or 100 % sequence identity with SEQ ID NO: **6.**

For the production of Ψ DP, an enzyme having nucleoside monophosphate kinase activity is needed which may be uridylate kinase (Ura6, SEQ ID NO: **8**) or an enzyme with at least 5, 10, 20, 30, 40, 45, 50, 55, 60, 65, 70, 75, 80, 85, 90, 95, 98, 99, 100 %, or an even higher specific activity of the nucleoside monophosphate kinase activity of SEQ ID NO: **8.** Alternatively, the enzyme having nucleoside monophosphate kinase activity is a functionally active variant of SEQ ID NO: **8** having at least 10, 20, 30, 40, 45, 50, 55, 60, 65, 70, 75, 80, 85, 90, 95, 98, 99, or 100 % sequence identity with SEQ ID NO: **8**.

For the production of Ψ TP, an enzyme having nucleoside diphosphate kinase activity is further added. This enzyme having nucleoside diphosphate kinase activity may be a nucleoside diphosphate kinase catalyzing the following reaction:

Ψ DP +ATP → ΨTP + ADP.

For the production of Ψ TP, a phosphoenol pyruvate kinase may be added as an enzyme having nucleoside diphosphate kinase activity catalyzing the following reaction:

PEP + Ψ DP → Pyr + Ψ TP.

According to one embodiment, an enzyme having nucleoside diphosphate kinase activity may be the pyruvate kinase PK given in SEQ ID NO: **11,** or an enzyme with at least 5, 10, 20, 30, 40, 45, 50, 55, 60, 65, 70, 75, 80, 85, 90, 95, 98, 99, 100 %, or an even higher specific activity of the nucleoside diphosphate kinase activity of SEQ ID NO: **11.** Alternatively, the enzyme having nucleoside diphosphate kinase activity is a functionally active variant of SEQ ID NO: **11** having at least 10, 20, 30, 40, 45, 50, 55, 60, 65, 70, 75, 80, 85, 90, 95, 98, 99, or 100 % sequence identity with SEQ ID NO: **11.**

For the production of Ψ TP, an acetyl phosphate kinase may be added as an enzyme having nucleoside diphosphate kinase activity catalyzing the following reaction:

AcP + Ψ DP → Ac + Ψ TP.

According to one embodiment, an enzyme having nucleoside diphosphate kinase activity may be acetyl phosphate kinase AcK given in SEQ ID NO: **7**, or an enzyme with at least 5, 10, 20, 30, 40, 45, 50, 55, 60, 65, 70, 75, 80, 85, 90, 95, 98, 99, 100 %, or an even higher specific activity of the nucleoside diphosphate kinase activity of SEQ ID NO: **7**. Alternatively, the enzyme having nucleoside diphosphate kinase activity is a functionally active variant of SEQ ID NO: **7** having at least 10, 20, 30, 40, 45, 50, 55, 60, 65, 70, 75, 80, 85, 90, 95, 98, 99, or 100 % sequence identity with SEQ ID NO: **7**.

For the production of Ψ TP, a polyphosphate kinase may be added as an enzyme having nucleoside diphosphate kinase activity catalyzing the following reaction:

PPPn + Ψ DP→ PPPn-1 + Ψ TP.

An enzyme having nucleoside diphosphate kinase activity may be the polyphosphate kinase MrPPK given in SEQ ID NO: **9**, or an enzyme with at least 5, 10, 20, 30, 40, 45, 50, 55, 60, 65, 70, 75, 80, 85, 90, 95, 98, 99, 100 %, or an even higher specific activity of the nucleoside diphosphate kinase activity of SEQ ID NO: **9.** Alternatively, the enzyme having nucleoside diphosphate kinase activity is a functionally active variant of SEQ ID NO: **9** having at least 10, 20, 30, 40, 45, 50, 55, 60, 65, 70, 75, 80, 85, 90, 95, 98, 99, or 100 % sequence identity with SEQ ID NO: **9.**

An enzyme having nucleoside diphosphate kinase activity may be the polyphosphate kinase Re_PPK2c given in SEQ ID NO: **10**, or an enzyme with at least 5, 10, 20, 30, 40, 45, 50, 55, 60, 65, 70, 75, 80, 85, 90, 95, 98, 99, 100 %, or an even higher specific activity of the nucleoside diphosphate kinase activity of SEQ ID NO: **10.** Alternatively, the enzyme having nucleoside diphosphate kinase activity is a functionally active variant of SEQ ID NO: **10** having at least 10, 20, 30, 40, 45, 50, 55, 60, 65, 70, 75, 80, 85, 90, 95, 98, 99, or 100 % sequence identity with SEQ ID NO: **10.**

Polyphosphate kinases for the phosphorylation of Ψ are described in literature [3, 4].

The activity of an enzyme can be determined by suitable methods for example, the phosphorylase activity of UP or any other enzyme having phosphorylase activity described herein is measured at 30° C in 100 mM phosphate buffer pH 7.5. About 0.05 µM UP are incubated with 0.2 mM uridine and the decrease in absorbance is measured. One unit of UP activity is the amount of enzyme producing 1 *µ* mol of uracil/min under the conditions used. See Vita, A. & Magni, G. (1983) as reference [8]. The Isomerase activity of DeoB or any other enzyme having isomerase activity described herein is measured at 30 ° in 50 mM HEPES buffer supplemented with 2 mM MnCl₂ (pH 7.0). About 180 *µ* M of DeoB is used together with excess of UP (110 µM) and YeiN (170 µM) in the reaction. The substrates are uridine (2 mM) and phosphate (1 mM). Samples taken are analyzes by HPLC. One unit of DeoB activity is the amount of enzyme producing 1 *µ* mol of D-Rib5P/min under the conditions used. The C-glycosidase activity of YeiN is measured at 30 ° in 50 mM HEPES buffer supplemented with 2 mM MnCl₂ (pH 7.0). About 1.5 *µ* M of YeiN is used in the reaction. The substrates are D-Rib5P (2 mM) and Ura (1mM). Samples taken are analyzes by HPLC. One unit of C-glycosidase activity is the amount of enzyme producing 1 *µ* mol of Ψ MP/min under the conditions used. See Pfeiffer, M., & Nidetzky, B. (2020) as reference [1]. The phosphatase activity of Yjjg, CIP, or any other enzyme having phosphatase activity described herein can be determined by incubation of 0.1-1 µM of enzyme at 30 ° C in 50 mM HEPES buffer supplemented with 1 mM MnCl₂ (pH 7.0) supplemented with 2 mM of substrate (Ψ MP). Inorganic phosphate is measured in samples at 850 nm. One unit of phosphatase activity is the amount of enzyme producing 1 *µ* mol of PO₄/min under the conditions used. See Saheki, S., Takeda, A. & Shimazu, T. (1985) as reference [9]. The kinase activity of an enzyme can be determined by incubation of 0.1-1 µM of enzyme at 30 ° C in 50 mM HEPES buffer supplemented with 1 mM MgCl₂ (pH 7.0), 2 mM of phosphate donor (ATP, AcP, polyphosphate, PEP, or any other suitable phosphate donor) and 2 mM phosphate acceptor (ADP, Ψ MP, Ψ DP, or any other suitable phosphate acceptor). Formation of the newly phosphorylated substrate is typically analyzed by HPLC. One unit of kinase activity is the amount of enzyme producing 1 *µ* mol of phosphorylated acceptor (ATP, Ψ DP, Ψ TP) /min under the conditions used.

According to one embodiment, the method of the invention may be performed as a one-pot reaction comprising an enzyme having phosphorylase activity, an enzyme having isomerase activity, and an enzyme having C-glycosidase activity.

According to one embodiment, the method of the invention may be performed as a one-pot reaction comprising an enzyme having phosphorylase activity, an enzyme having isomerase activity, an enzyme having C-glycosidase activity, and an enzyme having phosphatase activity.

According to a further embodiment, the one-pot reaction comprising an enzyme having phosphorylase activity, an enzyme having isomerase activity, and an enzyme having C-glycosidase activity, and an enzyme having phosphatase activity, wherein the enzyme having said phosphatase activity is Yjjg, or a functionally active variant thereof. This enzyme is able to remove the phosphate group from the C-nucleoside monophosphate in the course of the reaction, e.g., from pseudouridine monophosphate, while not dephosphorylating the substances ribose 1-phosphate and ribose 5-phosphate which are also present in the one-pot reaction as intermediate products. In this embodiment, recycling of the inorganic phosphate needed for the phosphorylase reaction is enabled directly in the one-pot reaction. This provides several advantages, namely the inhibition of the enzyme having isomerase activity by high phosphate concentration is reduced and simultaneously the corresponding C-nucleoside e.g., the pseudouridine, is produced which finally crystallizes from the reaction.

According to another embodiment of the invention, a one-pot reaction for the production of a C-nucleoside such as pseudouridine from uridine is performed using the following enzymes: UP, DeoB, YeiN, and Yjjg. Thereby, the enzymes UP, DeoB, YeiN, and Yjjg are added simultaneously to the reaction and catalyze the respective reactions simultaneously. In this specific case, a four enzymes cascade reaction is established.

According to an alternative embodiment, the method may be performed as two sequential reaction steps, wherein the first step of the one-pot reaction comprises the addition of an enzyme having phosphorylase activity, of an enzyme having isomerase activity, and of an enzyme having C-glycosidase activity. The second step comprises the addition of an enzyme having phosphatase activity. In this specific setup of the method provided herein, a C-nucleoside monophosphate is produced in the first reaction step and the corresponding C-nucleoside is produced in the second reaction step. In this case, e.g., acid phosphatases or alkaline phosphatases can be added for dephosphorylating the C-nucleotide such as the Ψ MP.

According to one embodiment, the method optionally further comprises the substitution of the N1 atom of the nucleobase of the produced C-nucleotide or C-nucleoside. Thereby, the C-nucleoside or the C-nucleotide produced by the method provided herein is subjected to a further modification reaction in order to substitute the N1 atom of the nucleobase. This modification reaction may performed be any method known to enable substitution of the N1 atom of the nucleobase e.g., by chemical or enzymatic modification.

According to a further embodiment. the C-nucleoside or the C-nucleotide produced by the method provided herein is subjected to a methylation reaction in order to produce a 1-N-methyl-C-nucleotide or a 1-N-methyl-C-nucleoside such as e.g., 1-N-methyl-pseudouridine-monophosphate or 1-N-methyl-pseudouridine.

According to one embodiment of the invention, 1-N-methyl-pseudouridine may be produced by methylation of pseudouridine. Such methylation of pseudouridine can be performed via chemical synthesis [5, 6, 7].

According to one embodiment of the invention, the phosphorylation of a C-nucleoside or C-nucleotide may be performed by chemical phosphorylation e.g., by chemical phosphorylation of Ψ [2].

According to another embodiment of the invention, an enzyme mixture is used for the production of a C-nucleotide, wherein said enzyme mixture comprises an enzyme having phosphorylase activity, an enzyme having isomerase activity, and an enzyme having C-glycosidase activity.

According to one embodiment of the invention, an enzyme mixture is used for the production of a C-nucleoside such as pseudouridine, wherein said enzyme mixture comprises an enzyme having phosphorylase activity, an enzyme having isomerase activity, an enzyme having C-glycosidase activity, and an enzyme having phosphatase activity. In a preferred embodiment, said enzyme having phosphatase activity is Yjjg.

According to one embodiment, the enzymes described herein or the enzyme mixture described herein may be in the form of different preparations known in the art. Non-limiting examples of such enzyme preparations are cell free extract, dried cell free extract, ammonium sulfate precipitate, lyophilizate, sterile filtered solution, sterilized solution, purified enzymes, and purified enzyme mixtures.

According to one embodiment of the invention, the method of the invention is performed at a suitable temperature, preferably at a temperature in the range of 4 to 90 ° C or even higher. Specifically, the method of the invention is performed at a temperature of 4, 5, 10, 15, 20, 21, 22, 23, 24, 25, 26, 27, 28, 29, 30, 31, 32, 33, 34, 35, 36, 37, 38, 39, 40, 45, 50, 55, 60, 65, 70, 75, 80, 85, 90 ° C, or even higher. Specifically, the method of the invention is performed at a temperature in the range of 20 to 40 ° C. Specifically, the method of the invention is performed at room temperature.

According to one embodiment of the invention, the N-nucleoside is provided in liquid or in solid form. The N-nucleoside may be dissolved in an aqueous or organic solution.

According to one embodiment of the invention, the method of the invention is performed in a suitable medium. Such a medium may be an aqueous solution, a buffer, or a buffered solution. In a preferred embodiment, the method of the invention is performed in phosphate buffer.

According to one embodiment of the invention, the products of the method described herein may be isolated and purified by standard operation means such as e.g., chromatography, precipitation, filtration, and/or crystallization.

According to one embodiment of the invention, the products produced by the method described herein are C-nucleosides or C-nucleotides. These products are produced from their corresponding N-nucleotides. For example, the C-nucleoside pseudouridine is produced from the corresponding N-nucleoside uridine.

According to one embodiment of the invention, the N-nucleoside is a pyrimidine nucleoside or a derivative thereof.

According to a preferred embodiment, the N-nucleoside is uridine, cytidine, or a derivative thereof.

According to a specific embodiment, the uridine derivative is of general formula I, 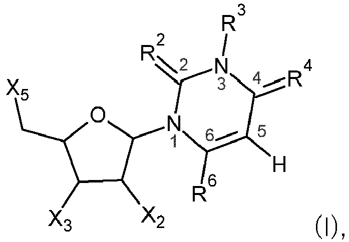 wherein
R²denotes O or S;
R³ denotes H, CH₃ or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
R⁴ denotes O or S;
R⁶denotes H, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X₂ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl; and
X₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl.

According to a specific embodiment, the cytidine derivative is of general formula II 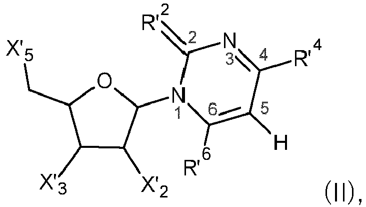 wherein
R'² denotes O or S;
R'⁴ denotes NH₂, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
R'⁶denotes H, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X'₂denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl;
X'₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl; and X'₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆Cycloalkylalkyl, C₆₋₁₀aryl, 3-8 membered heterocycloalkyl, and 5-12 membered heteroaryl.

According to one embodiment of the invention, the enzymes used in the method described herein may be naturally occurring, isolated enzymes or may be recombinantly produced.

According to one embodiment of the invention, the enzymes used in the method described herein or the enzyme mixture described herein may be expressed in different cells or co-expressed in one cell.

According to one embodiment, the cell co-expressing the enzymes described herein or the enzyme mixture described herein is used as a whole cell biocatalyst for performing the reactions or conversions described herein.

A whole cell biocatalyst is a whole cell, preferably a microorganism, comprising a catalyst, such as an enzyme, to perform chemical transformations on organic compounds. The enzyme may be located inside the cell or on the cell surface.

According to a specific embodiment, the whole cell biocatalyst may be a permeabilized whole cell, wherein the cell is treated with suitable methods and means for producing a permeabilized whole cell. For example, the cell may be treated with a suitable detergent or by freeze-thaw cycles.

According to another specific embodiment, a cell free extract comprising the enzymes used in the method described herein may be used to perform the method of the invention. Such a cell free extract may be produced by disrupting the cells producing the enzymes described herein. A cell free extract may be in liquid form or in dry form. A cell free extract may be in wet form. A cell free extract may be in lyophilized form.

According to another specific embodiment, the enzymes used in the methods described herein may be in the form of an ammonium sulfate precipitate.

The term "surface" in the term "surface of a cell" refers to any structure surrounding the cellular body of any of the known host cell. Such a structure is for example a plasma membrane. The term "plasma membrane" in connection with the present invention is to be understood as comprising any membrane and specifically, the extracellular surface of any such membrane.

According to one embodiment of the invention, the enzymes described herein may be recombinantly produced using a suitable host cell or may be recombinantly produced directly by the whole cell biocatalyst.

The cell co-expressing the enzymes described herein or the enzyme mixture described herein may be a prokaryotic or a eukaryotic cell, preferably a bacterial cell or a yeast cell. The cell may be an E. coli cell.

According to one embodiment of the invention, the genes encoding the enzymes described herein or the enzyme mixture described herein may be transformed into a suitable cell for recombinant expression or for use as a whole cell biocatalyst.

### Definitions

As used herein, the following definitions apply, unless stated otherwise:

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin ' s Genes XI", Jones & Bartlett Learning, (2017); Berg et al, "Stryer Biochemie" Springer Verlag, 2018; and Murphy & Weaver, "Janeway ' s Immunobiology" (9th Ed., or more recent editions), Taylor & Francis Inc, 2017.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges:
The "neutral" amino acids are shown below along with their respective three-letter and single-letter code and polarity: Alanine(Ala, A; nonpolar, neutral), Asparagine (Asn, N; polar, neutral), Cysteine (Cys, C; nonpolar, neutral), Glutamine (Gln, Q; polar, neutral), Glycine (Gly, G; nonpolar, neutral), Isoleucine (Ile, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).
The "positively" charged amino acids are: Arginine (Arg, R; polar, positive), and Lysine (Lys, K; polar, positive).
The "negatively" charged amino acids are: Aspartic acid (Asp, D; polar, negative), and Glutamic acid (Glu, E; polar, negative).

The term "nucleoside" refers to all compounds in which a heterocyclic base is covalently coupled to a sugar. The heterocyclic bases is also referred to as herein as a nucleobase. Therefore, in the present context the term "nucleoside" means the glycoside of a heterocyclic base. The term "nucleoside" may refer to naturally-occurring nucleosides, non-naturally occurring nucleosides, and other nucleoside analogs or derivatives. Illustrative examples of nucleosides are ribonucleosides comprising a ribose moiety as well as deoxyribonucleosides comprising a deoxyribose moiety. In addition, any modified variants of the naturally occurring sugar molecules found in nucleosides may be used. With respect to the bases of such nucleosides, it should be understood that this may be any of the naturally occurring bases e.g., adenine, guanine, cytosine, thymine, and uracil, as well as any modified variants thereof or any possible unnatural bases.

The term "nucleotide" refers to a nucleoside as defined herein further comprising a phosphate moiety. Nucleotides are composed of: a nucleobase, a sugar, and one or more phosphate group(s). Thereby, the phosphate group generally comprises one, two, or three phosphate group(s). Thus, a nucleotide may be a nucleoside-monophosphate, a nucleoside-diphosphate, or a nucleoside-triphosphate.

The term "N-nucleoside" refers to a nucleoside, wherein the coupling of the nucleobase to the sugar includes a C1'-(glycosidic) bond of a carbon atom in a sugar to a nitrogen in the nucleobase.

The term "C-nucleoside" refers to a nucleoside, wherein the coupling of the nucleobase to the sugar includes a C1'-(glycosidic) bond of a carbon atom in a sugar to a carbon in the heterocyclic base.

The term "C-nucleotide" refers to a C-nucleoside further comprising a phosphate group. Thereby, the phosphate group comprises one, two, or three phosphate group(s). Thus, the C-nucleotide may be a C-nucleoside-monophosphate, a C-nucleoside-diphosphate, or a C-nucleoside-triphosphate.

The term "corresponding" in the context of producing a C-nucleotide or C-nucleoside from the "corresponding N-nucleoside" as used herein refers to the C-nucleoside or C-nucleotide analog of said N-nucleoside. For example, the C-nucleoside pseudouridine is the C-nucleoside of the corresponding N-nucleoside uridine.

The term "sugar" refers to all carbohydrates and derivatives thereof, wherein in particular these derivatives may include deletion, substitution or addition of a chemical group or atom in the sugar. For example, deletions may include 2'-deoxy and/or 3'-deoxy sugars. For example, substitutions may include replacement of the ring-oxygen with sulphur or methylene, or replacement of a hydroxyl group with a halogen, an amino-, a sulfhydryl-, or a methyl group. For example, additions may include methylene phosphonate groups. Furthermore, the term "sugar" also includes sugar analogues i.e., not naturally occurring sugars. Modifications of the stereochemistry of a hydroxyl group is also covered herein such as e.g., the D- or L-conformation of the hydroxyl group of the C2 of C3 atom in a sugar molecule.

The term "sugar-phosphate moiety" as used herein refers to a sugar molecule with an attached phosphate group. According to a particular example, the sugar-phosphate moiety refers to a sugar with a phosphate group attached to the C1 atom of the sugar such as ribose 1-phosphate.

The term "providing" as used herein in the context of the method described herein refers to any operation or procedure to make the substrate of the described method available for the reaction to occur. This may encompass e.g., the addition of the N-nucleoside into a reactor or reaction vessel. Thereby, the N-nucleoside may be provided in solid or liquid form. The N-nucleoside may be dissolved before adding the enzymes described herein.

The term "enzyme" in accordance with the invention means any substance composed wholly or largely of protein or polypeptides that catalyzes or promotes, more or less specifically, one or more chemical or biochemical reaction(s).

The term "activity" as used herein in the context of enzymes or enzyme activity refers to the capability of an enzyme to catalyze a certain reaction. Enzyme activity can be defined in "specific activity" or "volumetric activity".

The term "unit" or "Unit" in the context of enzyme activity is defined as the following: 1 unit is the amount of enzyme that catalyzes the conversion of 1 µmol (micromole) of substrate per minute under the specified conditions of the assay method. Thus, 1 unit corresponds to 1 µmol/min.

The term "volumetric activity" or "volumetric enzyme activity" refers to the number of enzyme units per mL (Units/mL).

The term "specific activity" or "specific enzyme activity" refers to the number of enzyme units per mL divided by the concentration of the enzyme given in mg/mL. Specific enzyme activity is given in the unit "Units/mg".

The term "variant" or "functionally active variant" of an amino acid sequence or a nucleotide sequence as used herein means a sequence resulting from modification of this sequence by insertion, deletion or substitution of one or more amino acids or nucleotides within the sequence or at either or both of the distal ends of the sequence, and which modification does not affect (in particular impair) the activity of this sequence.

The term "one-pot" refers to a method of synthesis of chemical compounds through a single step in which the materials used are mixed together in a single vessel and allowed to react, rather than conducting the reaction in a sequence of separate stages. This strategy is used to improve reaction efficiency, increase yield and save time and resources.

As used herein, the term "mutation" as used herein has its ordinary meaning in the art. A mutation may comprise a point mutation, or refer to areas of sequences, in particular changing contiguous or non-contiguous amino acid sequences. Specifically, a mutation is a point mutation, which is herein understood as a mutation to alter one or more (but only a few) contiguous amino acids, e.g., 1, or 2, or 3 amino acids are substituted, inserted or deleted at one position in an amino acid sequence. Amino acid substitutions may be conservative amino acid substitutions or non-conservative amino acid substitutions. Conservative substitutions, as opposed to non-conservative substitutions, comprise substitutions of amino acids belonging to the same set or sub set, such as hydrophobic, polar, etc.

The term "variant" as used throughout the specification is to be understood to mean a nucleotide sequence of a nucleic acid or amino acid sequence of a protein or polypeptide that is altered by one or more nucleotides or amino acids, respectively. The variant may have "conservative" changes, wherein a substituted nucleotide or amino acid has similar structural or chemical properties to the replaced nucleotide or amino acid. A variant may also have "non-conservative" changes or a deletion and/or insertion of one or more nucleotides or amino acids. The term also includes within its scope any insertions/deletions of nucleotides or amino acids for a particular nucleic acid or protein or polypeptide. A "functional variant" will be understood to mean a variant that retains the functional capacity of a reference nucleotide sequence or a protein or polypeptide.

Functional variants may be obtained by sequence alterations in the polypeptide or the nucleotide sequence, e.g., by one or more point mutations, wherein the sequence alterations retain or improve a function of the unaltered polypeptide or the nucleotide sequence, when used in combination of the invention. Such sequence alterations can include, but are not limited to, (conservative) substitutions, additions, deletions, mutations and insertions. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

A point mutation is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids.

Specifically, a functionally active variant as described herein comprises at least 10, 20, 30, 40, 50, 60, 70, 80, 90, 100 % or even more of the enzymatic activity of the respective wild type enzyme or of the respective amino acid sequence to which the variant corresponds.

The term "sequence identity" as used herein is understood as the relatedness between two amino acid sequences or between two nucleotide sequences and described by the degree of sequence identity or sequence complementarity. The sequence identity of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searching is an effective and reliable strategy for identifying homologs with excess (e.g., at least 50%) sequence identity. Sequence similarity search tools frequently used are e.g., BLAST, FASTA, and HMMER.

Sequence similarity searches can identify such homologous proteins or polynucleotides by detecting excess similarity, and statistically significant similarity that reflects common ancestry. Homologues may encompass orthologues, which are herein understood as the same protein in different organisms, e.g., variants of such protein in different organisms or species.

"Percent (%) identity" with respect to an amino acid sequence, homologs and orthologues described herein is defined as the percentage of amino acid residues in a candidate sequence that are identical with the amino acid residues in the specific polypeptide sequence, after aligning the sequence and introducing gaps, if necessary, and not considering any conservative substitutions as part of the sequence identity. Those skilled in the art can determine appropriate parameters for the alignment, including any algorithms needed to achieve the highest scoring alignment over the full length of the sequences being compared. In case of percentages determined for sequence identities, it is possible that arithmetical decimal places may result which are not possible with regard to full nucleotides or amino acids. In this case, the percentages shall be rounded up to whole nucleotides or amino acids.

For purposes described herein, the sequence identity between two amino acid sequences is determined using standard methods, e.g., using the NCBI BLAST program version 2.2.29 (Jan-06-2014) or online using the multiple sequence alignment tool EMBL-EBI Clustal Omega (Sievers, F. et al. Fast, scalable generation of high-quality protein multiple sequence alignments using Clustal Omega. Mol. Syst. Biol. 7, 539 (2011)).

"Percent (%) identity" with respect to a nucleotide sequence e.g., of a nucleic acid molecule or a part thereof, in particular a coding DNA sequence, is defined as the percentage of nucleotides in a candidate DNA sequence that is identical with the nucleotides in the DNA sequence, after aligning the sequence and introducing gaps, if necessary, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for the alignment, including any algorithms needed to achieve the highest scoring alignment over the full length of the sequences being compared.

Optimal alignment may be determined with the use of any suitable algorithm for aligning sequences, non-limiting examples of which include the Smith-Waterman algorithm, the Needleman-Wunsch algorithm, MAFFT based algorithms: multiple alignment using fast Fourier transform, algorithms based on the Burrows-Wheeler Transform (e.g., the Burrows Wheeler Aligner), ClustalW, Clustal X, BLAT, Novoalign (Novocraft Technologies; available at novocraft.com), ELAND (Illumina, San Diego, CA), SOAP (available at soap.genomies.org.cn), and Maq (available at maq.sourceforge.net).

In a structure alignment the maximal set of corresponding pairs of amino acid residues that gives a good structural match when the structures are overlaid, i.e., superposed, is identified. Thereby, the positions of the protein's backbone C-alpha atoms and/or location of secondary structural elements are considered in this alignment. Tools for performing a structure alignment are available, e.g., the protein data bank provides a tool for pairwise structure alignment. Specifically, structure superposition is also a tool for determining corresponding amino acid positions in different enzymes. Structure superposition can be performed using the Molecular Graphics System PyMOL, (Schrödinger) using the command "align".

Unless specified otherwise, the term "alkyl", when used alone or in combination with other groups or atoms, refers to a saturated straight or branched chain consisting solely of 1 to 6 hydrogen-substituted carbon atoms, and includes methyl, ethyl, propyl, isopropyl, n-butyl, 1-methylpropyl, isobutyl, t-butyl, 2,2-dimethylbutyl, 2,2-dimethyl-propyl, n-pentyl, 2-methylpentyl, 3-methylpentyl, 4-methylpentyl, n-hexyl and the like.

Unless specified otherwise, the term "alkenyl" refers to a partially unsaturated straight or branched chain consisting solely of 2 to 6 hydrogen-substituted carbon atoms that contains at least one double bond, and includes vinyl, allyl, 2-methylprop-1-enyl, but-1-enyl, but-2-enyl, but-3-enyl, buta-1,3-dienyl, penta-1,3-dienyl, penta-2,4-dienyl, 2-methylbut-1-enyl, 2-methylpent-1-enyl, 4-methylpent-1-enyl, 4-methylpent-2-enyl, 2-methylpent-2-enyl, 4-methylpenta-1,3-dienyl, hexen-1-yl and the like.

Unless specified otherwise, the term "alkynyl" refers to a partially unsaturated straight or branched chain consisting solely of 2 to 6 hydrogen-substituted carbon atoms that contains at least one triple bond, and includes ethynyl, 1-propynyl, 2-propynyl, 2-methylprop-1-ynyl, 1-butynyl, 2-butynyl, 3-butynyl, 1,3-butadiynyl, 3-methylbut-1-ynyl, 4-methylbut-ynyl, 4-methylbut-2-ynyl, 2-methylbut-1-ynyl, 1-pentynyl, 2-pentynyl, 3-pentynyl, 4-pentynyl, 1,3-pentadiynyl, 1,4-pentadiynyl, 3-methylpent-1-ynyl, 4-methylpent-2-ynyl, 4-methylpent-2-ynyl, 1-hexynyl, and the like.

Unless specified otherwise, the term "cycloalkyl", when used alone or in combination with other groups or atoms, refers to monocyclic hydrocarbon rings, bicyclic hydrocarbon rings or spirohydrocarbon rings, which each may be either saturated or unsaturated (cycloalkenyl). The term unsaturated means that in the ring system in question there is at least one double bond, but no aromatic system is formed. In bicyclic hydrocarbon rings two rings are linked such that they have at least two carbon atoms in common. In spirohydrocarbon rings one carbon atom (spiroatom) is shared by two rings. If a cycloalkyl is substituted, the substitution may be mono- or polysubstitution in each case, at all the hydrogen-carrying carbon atoms, independently of one another. Cycloalkyl itself may be linked to the molecule as substituent via any suitable position of the ring system.

Typical examples of individual sub-groups are listed below. Monocyclic saturated hydrocarbon rings: cyclopropyl; cyclobutyl; cyclopentyl; cyclohexyl; cycloheptyl, etc.

Monocyclic unsaturated hydrocarbon rings: cycloprop-1-enyl; cycloprop-2-enyl; cyclobut-1-enyl; cyclobut-2-enyl; cyclopent-1-enyl; cyclopent-2-enyl; cyclopent-3-enyl; cyclohex-1-enyl; cyclohex-2-enyl; cyclohex-3-enyl; cyclohept-1-enyl; cyclohept-2-enyl; cyclohept-3-enyl; cyclohept-4-enyl; cyclobuta-1,3- dienyl; cyclopenta-1,4-dienyl; cyclopenta-1,3-dienyl; cyclopenta-2,4-dienyl; cyclohexa-1,3-dienyl; cyclohexa-1,5-dienyl; cyclohexa-2,4-dienyl; cyclohexa-1,4-dienyl; cyclohexa-2,5- dienyl, etc.
Saturated and unsaturated bicyclic hydrocarbon rings: bicyclo[2.2.0]hexyl; bicyclo[3.2.0]heptyl; bicyclo[3.2.1]octyl; bicyclo[2.2.2]octyl; bicyclo[4.3.0]nonyl (octahydroindenyl); bicyclo[4.4.0]decyl (decahydronaphthalene); bicyclo[2,2,1]heptyl (norbornyl); (bicyclo[2.2.1]hepta-2,5-dienyl (norborna-2,5-dienyl); bicyclo[2,2,1]hept-2-enyl (norbornenyl); bicyclo[4.1.0]heptyl (norcaranyl); bicyclo- [3.1.1]heptyl (pinanyl), etc.

Saturated and unsaturated spirohydrocarbon rings: spiro[2.5]octyl, spiro[3.3]heptyl, spiro[4.5]dec-2-ene, etc.

"Cycloalkylalkyl" denotes the combination of the above-defined groups alkyl, alkenyl, alkynyl, and cycloalkyl, in each case in their broadest sense. The alkyl group as substituent is directly linked to the molecule and is in turn substituted by a cycloalkyl group. The alkyl and cycloalkyl may be linked in both groups via any carbon atoms suitable for this purpose. The respective sub-groups of alkyl and cycloalkyl are also included in the combination of the two groups.

Unless specified otherwise, the term "aryl" refers to an aromatic mono- or bicyclic group containing from 6 to 14 carbon atoms, preferably 6 to 10 carbon atoms, that may be optionally fused with a fully or partially saturated or unsaturated carbocyclic ring and may optionally be substituted with one or more, identical or different substituents, suitably one to three substituents. Examples of aryl groups include phenyl, naphthyl, indanyl, and the like.

"Arylalkyl" denotes the combination of the groups alkyl, alkenyl, alkynyl and aryl as hereinbefore defined, in each case in their broadest sense. The alkyl group as substituent is directly linked to the molecule and is in turn substituted by an aryl group. The alkyl and aryl may be linked in both groups via any carbon atoms suitable for this purpose. Typical examples include benzyl, 1-phenylethyl, 2-phenylethyl, phenylvinyl, phenylallyl, etc.

Unless specified otherwise, the term "heteroaryl" refers to an aromatic mono- or bicyclic group containing from 5 to 14 carbon atoms, preferably 5 to 12 carbon atoms, of which one to five is replaced with a heteroatom selected from N, S and O, that may optionally be reduced to a non-aromatic heterocycle and may optionally be substituted with one or more, identical or different substituents. Examples of heteroaryl groups include pyrrolyl, dihydropyrrolyl, pyrrolidinyl, oxopyrrolidinyl, indolyl, isoindolyl, indolizinyl, imidazolyl, pyrazolyl, benzimidazolyl, imidazo(1,2-a)pyridinyl, indazolyl, purinyl, pyrrolo(2,3-c)pyridinyl, pyrrolo(3,2-c)pyridinyl, pyrrolo(2,3-b)pyridinyl, pyrazolo(1,5-a)pyridinyl, 1,2,3-triazolyl, 1,2,4-triazolyl, tetrazolyl, oxazolyl, 1,2 oxazolyl, isoxazolyl, 1,3,4-oxadiazolyl, 1,2,5-oxadiazolyl, 1,2,4-oxadiazolyl, 1,2,3-oxadiazolyl, thiazolyl, isothiazolyl, 1,3,4-thiadiazolyl, 1,2,5-thiadiazolyl, 1,2,4-thiadiazolyl, 1,2,3-thiadiazolyl, furanyl, dihydrofuranyl, tetrahydrofuranyl, benzofuranyl, isobenzofuranyl, thiophenyl, dihydrothiophenyl, tetrahydrothiophenyl, benzothiophenyl, benzoisothiophenyl, pyridyl, piperidinyl, quinolinyl, isoquinolinyl, tetrahydroisoqinolinyl, quinolizinyl, pyrazinyl, pyridazinyl, pyrimidinyl, pyranyl, tetrahydropyranyl, 1,2,3-triazinyl, 1,2,4-triazinyl, 1,3,5-triazinyl, chromenyl, morpholinyl, diazepinyl, benzodiazepinyl, and the like.

"Heteroarylalkyl" denotes the combination of the alkyl, alkenyl, alkynyl, and heteroaryl groups defined hereinbefore, in each case in their broadest sense. The alkyl group as substituent is directly linked to the molecule and is in turn substituted by a heteroaryl group. The linking of the alkyl and heteroaryl may be achieved on the alkyl side via any carbon atoms suitable for this purpose and on the heteroaryl side by any carbon or nitrogen atoms suitable for this purpose.

By the term "heterocycloalkyl" are meant groups which are derived from cycloalkyl as hereinbefore defined if in the hydrocarbon rings one or more of the groups -CH₂- are replaced independently of one another by the groups -O-, -S- or - NH- or one or more of the groups =CH- are replaced by the group =N-, while not more than five heteroatoms may be present in total, there must be at least one carbon atom between two oxygen atoms and between two sulphur atoms or between one oxygen and one sulphur atom and the group as a whole must be chemically stable. Heteroatoms may simultaneously be present in all the possible oxidation stages (sulphur -> sulphoxide -SO-, sulphone -SO₂-; nitrogen -> N-oxide). It is immediately apparent from the indirect definition/derivation from cycloalkyl that heterocycloalkyl is made up of the sub-groups monocyclic hetero-rings, bicyclic hetero-rings and spirohetero-rings, while each sub-group can also be further subdivided into saturated and unsaturated (heterocycloalkenyl). The term unsaturated means that in the ring system in question there is at least one double bond, but no aromatic system is formed. In bicyclic hetero-rings two rings are linked such that they have at least two atoms in common. In spirohetero-rings one carbon atom (spiroatom) is shared by two rings. If a heterocycloalkyl is substituted, the substitution may be mono- or polysubstitution in each case, at all the hydrogen-carrying carbon and/or nitrogen atoms, independently of one another. Heterocycloalkyl itself as substituent may be linked to the molecule via any suitable position of the ring system.

The term "heterocyclic group" as used herein refers to a heterocycloalkyl group which optionally may be fused to an aromatic aryl or heteroaryl group.

Typical examples of individual sub-groups are listed below: Monocyclic heterorings (saturated and unsaturated): oxolane, pyrrolidinyl, pyrrolinyl, imidazolidinyl, thiazolidinyl, imidazolinyl, pyrazolidinyl, pyrazolinyl, piperidinyl, piperazinyl, oxiranyl, aziridinyl, azetidinyl, 1,4-dioxanyl, azepanyl, diazepanyl, morpholinyl, thiomorpholinyl, homomorpholinyl, homopiperidinyl, homopiperazinyl, homothiomorpholinyl, thiomorpholinyl-S-oxide, thiomorpholinyl-S,S-dioxide, 1,3-dioxolanyl, oxane, tetrahydrothiopyranyl, 1,4-oxazepanyl, tetrahydrothienyl, homothiomorpholinyl-S,S-dioxide, oxazolidinonyl, dihydropyrazolyl, dihydropyrrolyl, dihydropyrazinyl, dihydropyridyl, dihydro-pyrimidinyl, dihydrofuryl, dihydropyranyl, tetrahydrothienyl-S-oxide, tetrahydrothienyl-S,S-dioxide, homothiomorpholinyl-S-oxide, 2,3-dihydroazet, 2*H-*pyrrolyl, 4*H*-pyranyl, 1,4- dihydropyridinyl, etc;
Bicyclic heterorings (saturated and unsaturated): 8-azabicyclo[3.2.1]octyl, 8-azabicyclo[5.1.0]octyl, 2-oxa-5-azabicyclo[2.2.1]heptyl, 8-oxa- 3-aza-bicyclo[3.2.1]octyl, 3,8-diaza-bicyclo[3.2.1]octyl, 2,5-diaza-bicyclo-[2.2.1]heptyl, 1-aza-bicyclo[2.2.2]octyl, 3,8-diaza-bicyclo[3.2.1]octyl, 3,9-diaza-bicyclo[4.2.1]nonyl, 2,6-diaza-bicyclo[3.2.2]nonyl, hexahydro-furo[3,2-b]furyl, etc;
Spiro-heterorings (saturated and unsaturated): 1,4-dioxa-spiro[4.5]decyl; 1-oxa-3,8-diaza-spiro[4.5]decyl; 2,6-diaza-spiro[3.3]heptyl; 2,7-diaza-spiro[4.4]nonyl; 2,6-diaza-spiro[3.4]octyl; 3,9-diaza-spiro[5.5]undecyl; 2,8-diaza- spiro[4.5]decyl, etc.

"Heterocycloalkylalkyl" denotes the combination of the alkyl, alkenyl, alkynyl, and heterocycloalkyl groups defined hereinbefore, in each case in their broadest sense. The alkyl group as substituent is directly linked to the molecule and is in turn substituted by a heterocycloalkyl group. The linking of the alkyl and heterocycloalkyl may be achieved on the alkyl side via any carbon atoms suitable for this purpose and on the heterocycloalkyl side by any carbon or nitrogen atoms suitable for this purpose.

By the term "suitable substituent" is meant a substituent that on the one hand is fitting on account of its valency and on the other hand leads to a system with chemical stability.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The term "tautomers" refer to compounds that are interchangeable forms of a particular compound structure, and that vary in the displacement of hydrogen atoms and electrons. Thus, two structures may be in equilibrium through the movement of *π* electrons and an atom (usually H). For example, enols and ketones are tautomers because they are rapidly interconverted by treatment with either acid or base.

It is also to be understood that compounds (e.g., dihydro bases described herein) that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively).

A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

It is understood by persons of ordinary skill in the art that polynucleotides, which encode one or more enzymes described herein for expression in the host cell or whole cell biocatalyst, can be operably linked to a promoter (to facilitate transcription), or operably linked to a signal sequence or leader peptide (to facilitate cellular processing and transport to the surface). Such genetic control elements and functional linkages thereto are numerous and well known in the art, and the present invention is not limited by the use thereof. Preferred promoters, however, include inducible promoters. Particularly preferred promoters (for eukaryotic systems) include those useful in yeast vectors, such as pGAL1, pGAL1-10, pGa1104, pGa110, pPGK, pCYC1, and pADH1.

The term "cell", "host cell", or "whole cell biocatalyst" as referred to herein is understood as any cell type that is susceptible to transformation, transfection, transduction, or the like with nucleic acid constructs or expression vectors comprising polynucleotides encoding expression products such as enzymes described herein, or susceptible to otherwise introduce any or each of the components of the enzymes described herein. Specifically, the host yeast cells are maintained under conditions allowing expression of the enzymes described herein.

Also described herein is a "cell line", a "host cell line" or a "production cell line", which is commonly understood to be a cell line ready-to-use for cultivation/culturing in a bioreactor to obtain the product of an expression production process, such as the enzyme as described herein, or to obtain the product of the production process, such as a C-nucleoside or C-nucleotide as described herein. The cell, cell line, or the whole cell biocatalyst cell line as described herein is particularly understood as a recombinant microorganism, which may be cultivated/cultured to produce the desired compound.

The term "cell culture" or "cultivation" ("culturing" is herein synonymously used), also termed "fermentation", with respect to a host cell line is meant to be the maintenance of cells in an artificial, e.g., an *in vitro* environment, under conditions favoring growth, differentiation or continued viability, in an active or quiescent state, of the cells, specifically in a controlled bioreactor according to methods known in the industry. When cultivating, a cell culture is brought into contact with the cell culture media in a culture vessel or with substrate under conditions suitable to support cultivation of the cell culture. In certain embodiments, a culture medium as described herein is used to culture cells according to standard cell culture techniques that are well-known in the art for cultivating or growing cells.

Expression products such as polypeptides, proteins or protein domains, or RNA molecules as described herein, including e.g., the enzymes as described herein, may be introduced into a host cell either by introducing the respective coding polynucleotide or nucleotide sequence for expressing the expression products within the host cell, or by introducing the respective expression products which are within an expression system or isolated.

Any of the known procedures for introducing expression cassettes, vectors or otherwise introducing (e.g., coding) nucleotide sequences into host cells may be used. These include the use of calcium phosphate transfection, polybrene, protoplast fusion, electroporation, nucleofection, liposomes, microinjection, naked DNA, plasmid vectors, viral vectors, both episomal and integrative, and any of the other well-known methods for introducing cloned genomic DNA, cDNA, synthetic DNA or other foreign genetic material into a host cell (see, e.g., Sambrook et al.).

The term "expression" as used herein regarding expressing a polynucleotide or nucleotide sequence, is meant to encompass at least one step selected from the group consisting of DNA transcription into mRNA, mRNA processing, non-coding mRNA maturation, mRNA export, translation, protein folding and/or protein transport. Nucleic acid molecules containing a desired nucleotide sequence may be used for producing an expression product encoded by such nucleotide sequence e.g., proteins or transcription products such as RNA molecules, in particular fusion proteins as described herein. To express a desired nucleotide sequence, an expression system is conveniently used, which can be an *in vitro* or *in vivo* expression system, as necessary to express a certain nucleotide sequence by a host cell or host cell line. Typically, host cells are transfected or transformed with an expression system comprising an expression cassette that comprises the desired nucleotide sequence and a promoter operably linked thereto optionally together with further expression control sequences or other regulatory sequences. Specific expression systems employ expression constructs such as vectors comprising one or more expression cassettes.

The term "expression construct" as used herein, means the vehicle, e.g. vectors or plasmids, by which a DNA sequence is introduced into a host cell so as to transform the host and promote expression (e.g. transcription and translation) of the introduced sequence. "Expression construct" as used herein includes both, autonomously replicating nucleotide sequences as well as genome integrating nucleotide sequences.

The terms "vector", "DNA vector" and "expression vector" mean the vehicle by which a DNA sequence (e.g. a foreign gene) can be introduced into a host cell, so as to transform the host and promote expression (e.g. transcription and translation) of the introduced sequence. Vector" as used herein includes both, autonomously replicating nucleotide sequences as well as genome integrating nucleotide sequences, such as artificial chromosomes. Plasmids are preferred vectors of the invention.

In specific embodiments, an expression vector may contain more than one expression cassettes, each comprising at least one coding sequence and a promoter in operable linkage.

A "cassette" refers to a DNA coding sequence or segment of DNA that codes for an expression product that can be inserted into a vector at defined restriction sites. The cassette restriction sites are designed to ensure insertion of the cassette in the proper reading frame. An "expression cassette" as used herein refers to nucleic acid molecules containing a desired coding sequence and control sequences in operable linkage, so that an expression system can use such expression cassette to produce the respective expression products, including *e*.*g*., encoded proteins or other expression products. Certain expression systems employ host cells or host cell lines which are transformed or transfected with an expression cassette, which host cells are then capable of producing expression products *in vivo.* In order to effect transformation of host cells, an expression cassette may be conveniently included in a vector, which is introduced into a host cell; however, the relevant DNA may also be integrated into a host chromosome. A coding sequence is typically a coding DNA or coding DNA sequence which encodes a particular amino acid sequence of a particular polypeptide or protein, or which encodes any other expression product.

The term "expression vector" means a linear or circular DNA molecule that comprises a polynucleotide encoding a polypeptide and is operably linked to control sequences that provide for its expression. Vectors typically comprise DNA sequences that are required for the transcription of cloned recombinant nucleotide sequences, *i.e.* of recombinant genes and the translation of their mRNA in a suitable host organism. A coding DNA sequence or segment of DNA molecule coding for an expression product can be conveniently inserted into a vector at defined restriction sites. To produce a vector, heterologous foreign DNA can be inserted at one or more restriction sites of a vector DNA, and then is carried by the vector into a host cell along with the transmissible vector DNA. It is preferred that a vector comprises an expression system, *e*.*g*. one or more expression cassettes. Expression cassette restriction sites are designed to ensure insertion of the cassette in the proper reading frame.

To obtain expression, a sequence encoding a desired expression product, such as *e.g.* any of the polypeptides, proteins or protein domains described herein, is typically cloned into an expression vector that contains a promoter to direct transcription. Appropriate expression vectors typically comprise regulatory sequences suitable for expressing coding DNA. Examples of regulatory sequences include promoter, operators, enhancers, ribosomal binding sites, and sequences that control transcription and translation initiation and termination. The regulatory sequences are typically operably linked to the DNA sequence to be expressed.

A promoter is herein understood as a DNA sequence which initiates, regulates, or otherwise mediates or controls the expression of the coding DNA. Promoter DNA and coding DNA may be from the same gene or from different genes, and may be from the same or different organisms. Recombinant cloning vectors often include one or more replication systems for cloning or expression, one or more markers for selection in the host, e.g., antibiotic resistance, one or more nuclear localization signals (NLS) and one or more expression cassettes.

### Examples

The Examples which follow are set forth to aid in the understanding of the invention but are not intended to, and should not be construed to, limit the scope of the invention in any way. The Examples do not include detailed descriptions of conventional methods. Such methods are well known to those of ordinary skill in the art.

### GENERAL EXPERIMENTAL DETAILS

### MATERIALS

The enzymes referred to as herein are given in the following table 1 (Ψ : pseudouridine, Ψ MP: pseudouridine-monophosphate, Ψ DP: pseudouridine-diphosphate, Ψ TP: pseudouridine-triphosphate).

**Table 1**

| **Seq ID NO:** | **Enzyme** | **UniProt_Entry** | **EC** | **reactions** |
|---|---|---|---|---|
| 1 | UP | P12758 | EC:2.4.2.3 | uridine→ Ura+ Rib1P |
| 2 | PNP | P0C037 | EC:2.4.2.1 | uridine→ Ura+ Rib1P |
| 3 | DeoB | P0A6K6 | EC:5.4.2.7 | Rib1P → Rib5P |
| 4 | YeiN | Q1R9Q7 | EC:4.2.1.70 | Rib5P + Ura → ΨMP |
| 13 | IndA | Q9X1H5 | EC:4.2.1.70 | Rib5P + Ura → ΨMP |
| 14 | OzmD | | EC:4.2.1.70 | Rib5P + Ura → ΨMP |
| 15 | SdmA | | EC:4.2.1.70 | Rib5P + Ura → ΨMP |
| 16 | MinB | | EC:4.2.1.70 | Rib5P + Ura → ΨMP |
| 5 | Yjjg | P0A8Y1 | EC:3.1.3.5 | Ψ MP + H₂O→ Ψ + PO₄ |
| 6 | CMPK | C3TGB7 | EC:2.7.4.25 | Ψ MP + ATP → ΨDP + ADP |
| 7 | AcK | P38502 | EC:2.7.2.1 | AcP + ADP → Ac +ATP AcP + Ψ DP → Ac + Ψ TP |
| 8 | Ura6 | P15700 | EC:2.7.4.14 | ΨMP + ATP → ΨDP + ADP |
| 9 | MrPPK | M9XB82 | EC:2.7.4.33 | PPPₙ + ADP → PPPn₋₁ +ATP PPPₙ + ΨDP → PPPₙ₋₁ + Ψ TP |
| 10 | Re_PPK2c | Q0KCB8 | EC:2.7.4.33 | PPPₙ + ADP → PPPₙ₋₁ +ATP PPPₙ + ΨDP → PPPₙ₋₁ + Ψ TP |
| 11 | PK | P11974 | EC:2.7.1.40 | PEP + ADP → Pyr +ATP PEP + ΨDP → Pyr Ψ TP |
| 12 | CIP | P19111 | EC:3.1.3.1 | R-PO₃ + H₂O→ R-OH + PO₄ |

The amino acid sequences of these enzymes are given in the following:
SEQ ID NO: 1 - EcUP:
   >sp|P12758.31UDP_ECOLI RecName: Full=Uridine phosphorylase; Short=UPase; Short= UrdPase 
MSKSDVFHLGLTKNDLQGATLAIVPGDPDRVEKIAALMDKPVKLASHREFTTWRAEL
DGKPVIVCSTGIGGPSTSIAVEELAQLGIRTFLRIGTTGAIQPHINVGDVLVTTASVRLDG
ASLHFAPLEFPAVADFECTTALVEAAKSIGATTHVGVTASSDTFYPGQERYDTYSGRVV
RHFKGSMEEWQAMGVMNYEMESATLLTMCASQGLRAGMVAGVIVNRTQQEIPNAET
MKQTESHAVKIVVEAARRLL
SEQ ID NO: 2 - EcPNP:

   >sp|P0C037.1|PPNP_ECOLI RecName: Full=Pyrimidine/purine nucleoside phosphorylase; AltName: Full=Adenosine phosphorylase; AltName: Full=Cytidine phosphorylase; AltName: Full=Guanosine phosphorylase; AltName: Full=Inosine phosphorylase; AltName: Full=Thymidine phosphorylase; AltName: Full=Uridine phosphorylase; AltName: Full=Xanthosine phosphorylase 
MLQSNEYFSGKVKSIGFSSSSTGRASVGVMVEGEYTFSTAEPEEMTVISGALNVLLPD
ATDWQVYEAGSVFNVPGHSEFHLQVAEPTSYLCRYL
SEQ ID NO: 3 - DeoB:
   >sp|P0A6K6.1|DEOB_ECOLI RecName: Full=Phosphopentomutase; AltName: Full = Phosphodeoxyribomutase 
MKRAFIMVLDSFGIGATEDAERFGDVGADTLGHIAEACAKGEADNGRKGPLNLPNLTR
LGLAKAHEGSTGFIPAGMDGNAEVIGAYAWAHEMSSGKDTPSGHWEIAGVPVLFEWG
YFSDHENSFPQELLDKLVERANLPGYLGNCHSSGTVILDQLGEEHMKTGKPIFYTSAD
SVFQIACHEETFGLDKLYELCEIAREELTNGGYNIGRVIARPFIGDKAGNFQRTGNRHDL
AVEPPAPTVLQKLVDEKHGQVVSVGKIADIYANCGITKKVKATGLDALFDATIKEMKEA
GDNTIVFTNFVDFDSSWGHRRDVAGYAAGLELFDRRLPELMSLLRDDDILILTADHGC
DPTWTGTDHTREHIPVLVYGPKVKPGSLGHRETFADIGQTLAKYFGTSDMEYGKAMF
SEQ ID NO: 4-YeiN:
   >sp|Q1R9Q7|Q1R9Q7_ECOUT Hypothetical protein YeiN 
MSELTLSPELLQISAEVQDALKNKKSVVALESTIISHGMPFPQNAQTAIEVEETIRKQGA
VPATIAIIGGVMKVGLSKEEIELLGREGHNVTKVSRRDLPFVVAAGKNGATTVASTMIIA
ALAGIKVFATGGIGGVHRGAEHTFDISADLQELANTNVTVVCAGAKSILDLGLTTEYLET
FGVPLIGYQTKALPAFFCRTSPFDVSIRLDSAREIARAMAVKWQTGLNGGLVVANPIPE
QFAMPEETINAAIDQAVAEAEEQGVIGKESTPFLLARVAELTGGDSLKSNIQLVFNNAIL
ASEIAKEYQRLVG
SEQ ID NO: 5 - Yjjg:
   >sp|P0A8Y1.1|YJJG_ECOLI RecName: Full=Pyrimidine 5'-nucleotidase YjjG; AltName: Full=House-cleaning nucleotidase; AltName: Full=Non-canonical pyrimidine nucleotide phosphatase; AltName: Full=Nucleoside 5'-monophosphate phosphohydrolase; AltName: Full=dUMP phosphatase 
MKWDWIFFDADETLFTFDSFTGLQRMFLDYSVTFTAEDFQDYQAVNKPLWVDYQNG
AITSLQLQHGRFESWAERLNVEPGKLNEAFINAMAEICTPLPGAVSLLNAIRGNAKIGIIT
NGFSALQQVRLERTGLRDYFDLLVISEEVGVAKPNKKIFDYALEQAGNPDRSRVLMVG
DTAESDILGGINAGLATCWLNAHHREQPEGIAPTWTVSSLHELEQLLCKH
SEQ ID NO: 6 - cytidylate kinase:
   >CCQ27868.2 cytidylate kinase [Escherichia coli] 
MTAIAPVITIDGPSGAGKGTLCKAMAEALQWHLLDSGAIYRVLALAALHHHVDVASED
ALVPLASHLDVRFVSTNGNLEVILEGEDVSGEIRTQEVANAASQVAAFPRVREALLRRQ
 
RAFRELPGLIADGRDMGTVVFPDAPVKIFLDASSEERAHRRMLQLQEKGFSVNFERLL
AEIKERDDRDRNRAVAPLVPAADALVLDSTTLSIEQVIEKALQYARQKLALA
SEQ ID NO: 7-AcK:
   >sp|P38502.1|ACKA_METTE RecName: Full=Acetate kinase; AltName: Full=Acetokinase 
MKVLVINAGSSSLKYQLIDMTNESALAVGLCERIGIDNSIITQKKFDGKKLEKLTDLPTH
KDALEEVVKALTDDEFGVIKDMGEINAVGHRVVHGGEKFTTSALYDEGVEKAIKDCFEL
APLHNPPNMMGISACAEIMPGTPMVIVFDTAFHQTMPPYAYMYALPYDLYEKHGVRK
YGFHGTSHKYVAERAALMLGKPAEETKIITCHLGNGSSITAVEGGKSVETSMGFTPLEG
LAMGTRCGSIDPAIVPFLMEKEGLTTREIDTLMNKKSGVLGVSGLSNDFRDLDEAASK
GNRKAELALEIFAYKVKKFIGEYSAVLNGADAVVFTAGIGENSASIRKRILTGLDGIGIKID
DEKNKIRGQEIDISTPDAKVRVFVIPTNEELAIARETKEIVETEVKLRSSIPV
SEQ ID NO: 8 - Ura6:
   >sp|P15700.1|KCY_YEAST RecName: Full=Uridylate kinase; Short=UK; AltName: Full=ATP:UMP phosphotransferase; AltName: Full=Deoxycytidylate kinase; Short=CK; Short=dCMP kinase; AltName: Full=Suppressor of cdc8 protein; AltName: Full=Uridine monophosphate kinase; Short=UMP kinase; Short=UMPK 
MTAATTSQPAFSPDQVSVIFVLGGPGAGKGTQCEKLVKDYSFVHLSAGDLLRAEQGRA
GSQYGELIKNCIKEGQIVPQEITLALLRNAISDNVKANKHKFLIDGFPRKMDQAISFERDI
VESKFILFFDCPEDIMLERLLERGKTSGRSDDNIESIKKRFNTFKETSMPVIEYFETKSK
VVRVRCDRSVEDVYKDVQDAIRDSL
SEQ ID NO: 9 - MrPPK:

   >sp|M9XB82.1|PK23_MEIRD RecName: Full=AMP/ADP-polyphosphate phosphotransferase 
MKKYRVQPDGRFELKRFDPDDTSAFEGGKQAALEALAVLNRRLEKLQELLYAEGQHK
VLVVLQAMDAGGKDGTIRVVFDGVNPSGVRVASFGVPTEQELARDYLWRVHQQVPRK
GELVIFNRSHYEDVLVVRVKNLVPQQVWQKRYRHIREFERMLADEGTTILKFFLHISKD
EQRQRLQERLDNPEKRWKFRMGDLEDRRLWDRYQEAYEAAIRETSTEYAPWYVIPAN
KNWYRNWLVSHILVETLEGLAMQYPQPETASEKIVIE
SEQ ID NO: 10 - Re_PPK2c:
   >sp|Q0KCB8|Q0KCB8_RALEU Hypothetical protein h16_A1212 [Cupriavidus necator H16] 
MTDRDTIQSSHTATAPRARATARGDVLPTNSALSAQRNEVDSAVDASVQLAASSMQDI
LASRAGNGADMLDAMRTLLDGLAPDEAAQLRSLILEGDPAAWQAGRKRHPDDELSAG
WREGAYPYQNLMSRRNYEKQKYRLQVELLKFQAWVRETGQRVVIVFEGRDAAGKGGT
IKRFMEHMNPRGARVVALEKPTEAERGQWYFQRYVQHLPSAGEIVLFDRSWYNRAGV
EHVMGFCSQREYQDFLQQAPDFERHLVRSGIHLFKFWFSVSQKEQRRRFREREIHPLK
QWKLSPVDLASLDKWDEYTQAKEAMFAHTDTADAPWTVIRSDCKKRARLNALRAILS
RFPYGNRDTTAIGQPDPLIVGRALAN
SEQ ID NO: 11 - PK:
   >sp|P11974.41KPYM_RABIT RecName: Full=Pyruvate kinase PKM; AltName: Full=Pyruvate kinase muscle isozyme 
MSKSHSEAGSAFIQTQQLHAAMADTFLEHMCRLDIDSAPITARNTGIICTIGPASRSVET
LKEMIKSGMNVARMNFSHGTHEYHAETIKNVRTATESFASDPILYRPVAVALDTKGPEI
RTGLIKGSGTAEVELKKGATLKITLDNAYMEKCDENILWLDYKNICKVVDVGSKVYVD
DGLISLQVKQKGPDFLVTEVENGGFLGSKKGVNLPGAAVDLPAVSEKDIQDLKFGVEQ
DVDMVFASFIRKAADVHEVRKILGEKGKNIKIISKIENHEGVRRFDEILEASDGIMVARG
DLGIEIPAEKVFLAQKMIIGRCNRAGKPVICATQMLESMIKKPRPTRAEGSDVANAVLD
GADCIMLSGETAKGDYPLEAVRMQHLIAREAEAAMFHRKLFEELARSSSHSTDLMEA
MAMGSVEASYKCLAAALIVLTESGRSAHQVARYRPRAPIIAVTRNHQTARQAHLYRGIF
PVVCKDPVQEAWAEDVDLRVNLAMNVGKARGFFKKGDVVIVLTGWRPGSGFTNTMR
VVPVP
SEQ ID NO: 12-CIP:
   >sp|P19111.2|PPBI_BOVIN RecName: Full=Intestinal-type alkaline phosphatase; Short=IAP; Short=Intestinal alkaline phosphatase; Flags: Precursor 
MQGACVLLLLGLHLQLSLGLVPVEEEDPAFWNRQAAQALDVAKKLQPIQTAAKNVILF
LGDGMGVPTVTATRILKGQMNGKLGPETPLAMDQFPYVALSKTYNVDRQVPDSAGT
ATAYLCGVKGNYRTIGVSAAARYNQCKTTRGNEVTSVMNRAKKAGKSVGVVTTTRVQ
HASPAGAYAHTVNRNWYSDADLPADAQMNGCQDIAAQLVNNMDIDVILGGGRKYMF
PVGTPDPEYPDDASVNGVRKRKQNLVQAWQAKHQGAQYVWNRTALLQAADDSSVT
HLMGLFEPADMKYNVQQDHTKDPTLQEMTEVALRVVSRNPRGFYLFVEGGRIDHGH
HDDKAYMALTEAGMFDNAIAKANELTSELDTLILVTADHSHVFSFGGYTLRGTSIFGLA
PSKALDSKSYTSILYGNGPGYALGGGSRPDVNDSTSEDPSYQQQAAVPQASETHGGE
DVAVFARGPQAHLVHGVEEETFVAHIMAFAGCVEPYTDCNLPAPTTATSIPDAAHLAA
SPPPLALLAGAMLLLLAPTLY
SEQ ID NO: 13 - IndA
   >sp|Q9X1H5|PSUG_THEMA Pseudouridine-5'-phosphate glycosidase OS=Thermotoga maritima (strain ATCC 43589 / DSM 3109 / JCM 10099 / NBRC 100826 / MSB8) OX=243274 GN=psuG PE=1 SV=1 
MIIESRIEKGKPVVGMETTVFVHGLPRKEAIELFRRAKEISREKGFQLAVIGILKGKIVAG
MSEEELEAMMREGADKVGTREIPIVVAEGKNAATTVSATIFLSRRIGIEVVVTGGTGGVH
PGRVDVSQDLTEMSSSRAVLVSSGIKSILDVEATFEMLETLEIPLVGFRTNEFPLFFSRK
SGRRVPRIENVEEVLKIYESMKEMELEKTLMVLNPVPEEYEIPHDEIERLLEKIELEVEG
KEVTPFLLKKLVEMTNGRTLKANLALLEENVKLAGEIAVKLKRS
SEQ ID NO: 14-OzmD
   >WP_185036055.1 pseudouridine-5'-phosphate glycosidase [Streptomyces candidus] 
MTRSSSLPPRSVPHTPIPLVYSDEVAEALHAGDPVVALESNVITHGLPYPDNAATARKV
EAAVRTGGAVPATIGVDEGRLLIGLSEDDIERFASTPGIPKVSSRDLPVVLARGGKGATT
VASSLVAAELAGLPFFASAGIGGVHRGAQATMDISSDLVQFTRSKVAVVCAGAKNILDL
GLTMEYLETQCVPLLSYRSDDFPAFYCASSGIRSPHRIDDEEVIARVIENHWATGSNSS
VVITTPPRPEDAVDSEVAEAALREALADAERDGIRGNALTKYLMRAIDRTTGGRTSQAN
MAVLISTAETGGRLAAAHARLRREAAAQ
SEQ ID NO: 15 - SdmA:
   >KKZ73237.1 pseudouridine-5'-phosphate glycosidase [Streptomyces showdoensis] 
MTTSSHLRAPAFDPTLLALSEEVSEALAHGRPVVALESSVLSGFPYPENLELAREMDA
GIRAQGAVPARIALLGGKLRVGLDDALLQEFCAYGKVPKVSTRDIGVTLAGGGPGGTTV
SSSLLAAELAGIRVFAVAGIGGVHRDAPASYDISTDLVQFTRTRTAVVCAGAKSMLHPQ
WTLEWLETAGIPVIGYRCDDFPAYLSVSSGVRNPHRLDDLSDVARATWAHWRSGNGT
SVLVTHPIAEDDGIPSQEIADALAEATELAAREGITGPAVTPYLLKAIAEATEGRTAAANR
SVLKSTTVLAAEFAVHLAKEEQR
SEQ ID NO: 16 - MinB:
   >AFV27435.1 IndA [Streptomyces chromofuscus]

MTIPAPRHRSVPLVFTDEVAAALADGDPVVALESNVITHGLPYPDNAATARKVEDAVR
AGGAVPATIGIDDGRIMVGMTDGDIERFAATPGIPKVSSRDLAAVLASGGTGATTVASS
LVAAELAGIPFFSSAGIGGVHRGAETTMDISSDLIQFTRSRVAVVCAGAKKILDLGLTLEF
LETHCVPVIAHGSDDFPAFYCRSSGLRAPQRLDDETLIARAVETHWALGNPGGVLVTS
PTRPEDAIDEQDVEGAIADALRGAEADGVRGGAVTKYVMRAVDRATEGRSARANTAVL
IHTAQIGGRIAAAHARLRREEDQ

### Example 1 - One pot multi enzyme cascade synthesis of pseudouridine-monophosphate (ΨMP)

### SCHEME 1: One pot multi-enzyme cascade synthesis of ΨMP.

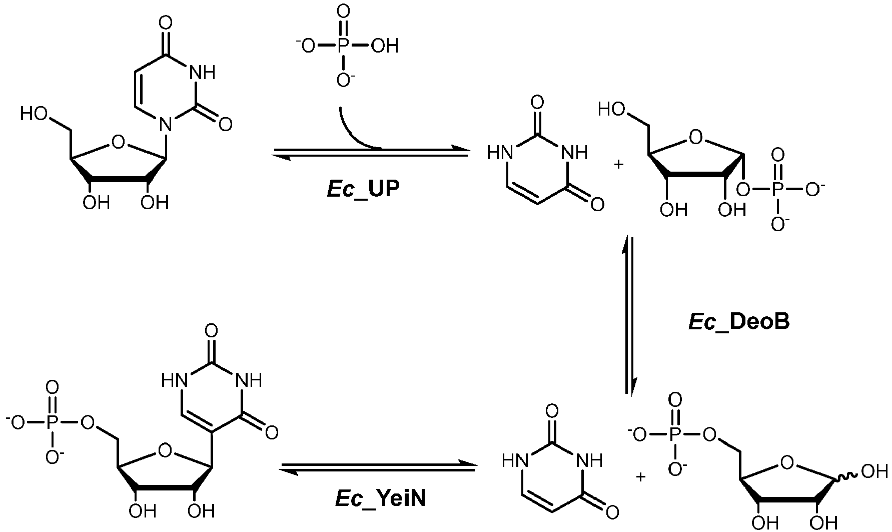

### Synthesis of 1g ΨMP:

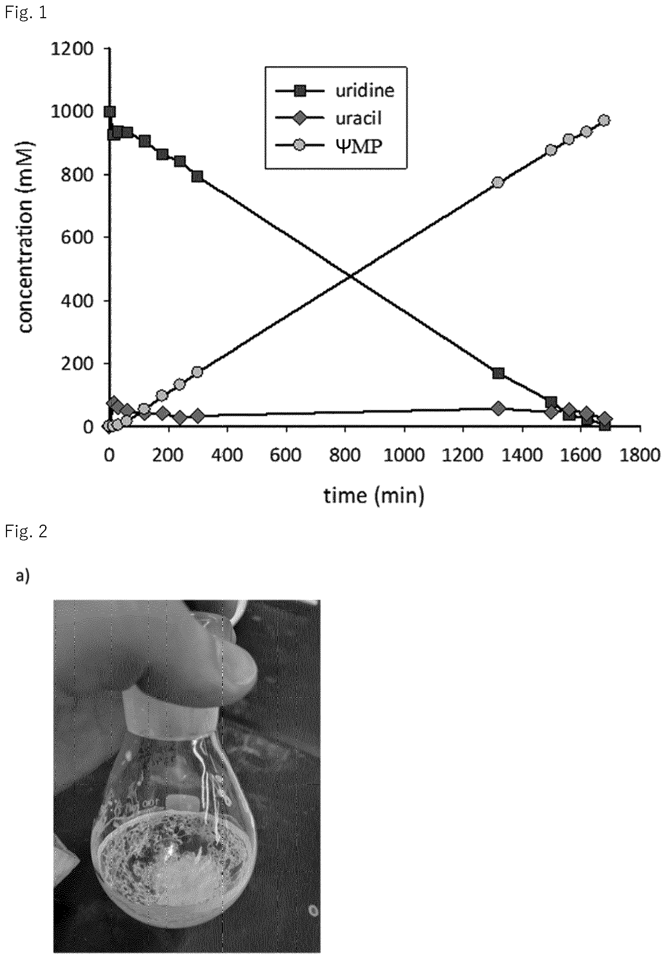

Synthesis was performed in 5 mL scale and 1 M KH₂PO₄, 1 M uridine, 20 mM MnCl₂, were dissolved in water and the pH was set to 7.0 with KOH. The reaction was initiated by addition of 0.25 mg/mL Ec_UP, 2.5 mg/mL Ec_DeoB, 1.5 mg/mL EC_YeiN and incubated at 30° C for 30 h. After 28 h synthesis was completed with > 97 % conversion. Enzymes were heat inactivated (99 ° C for 7 min) and removed by centrifugation. The time course of Ψ MP synthesis is shown in Fig. 1.

### ΨMP isolation:

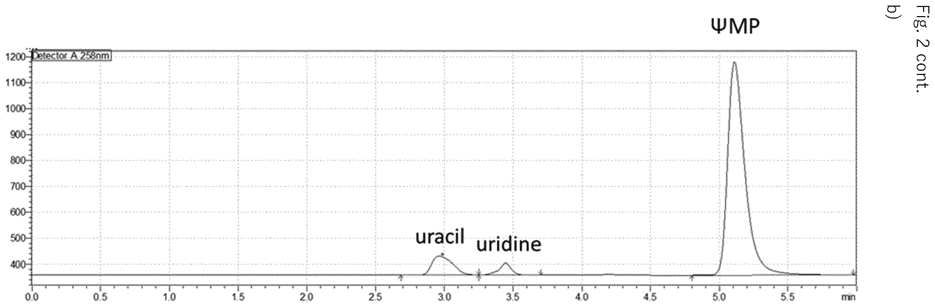

Residual inorganic phosphate was precipitated by addition of equimolar BaOH and removed by centrifugation. The cleared supernatant was lyophilized and yielded 1.6 g of ΨMP (> 95% yield). ΨMP after lyophilization is shown in Fig. 2 a). HPLC analysis of isolated ΨMP is shown in Fig. 2 b).
Following centrifugation, barium phosphate crystals were removed and the supernatant was subjected to lyophilization. The resulting yellowish powder was analyzed by NMR and the spectra agree well with previously published data [1]. The annotated NMR results of the product are given in the following: ¹H NMR (300 MHz, Deuterium Oxide) of ΨMP: δ 7.83 (s, H-6, 1H), 4.69 (s, H-1', 1H), 4.18 (dd, J = 6.7, 4.8 Hz, H-3', 1H), 4.10 (t, J = 4.3 Hz, H-4', 1H), 3.97 (dt, J = 6.7, 2.9 Hz, 1H), 3.93 - 3.71 (m, H-5', 2H).

¹³C NMR (76 MHz, Deuterium Oxide) of ΨMP: δ 166.40 (C-2), 154.27 (C-4), 142.36 (C-6), 111.41 (C-5), 81.51 (C-4'), 78.57 (C-1'), 74.46 (C-2'), 69.81 (C-3'), 62.72 (C-5').

### Example 2 - One pot multi enzyme cascade synthesis of pseudouridine (Ψ) SCHEME 2: One pot multi-enzyme cascade synthesis of Ψ.

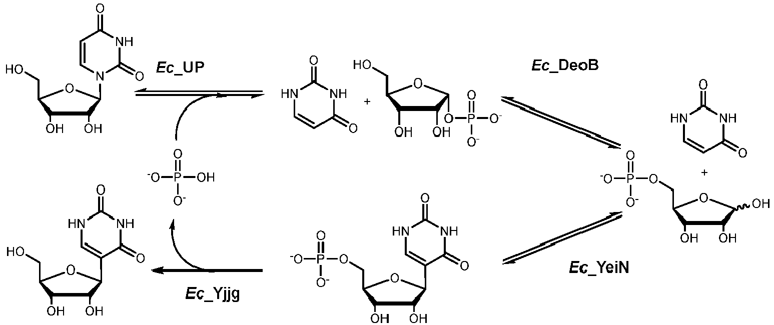

### Synthesis of 1 g Ψ:

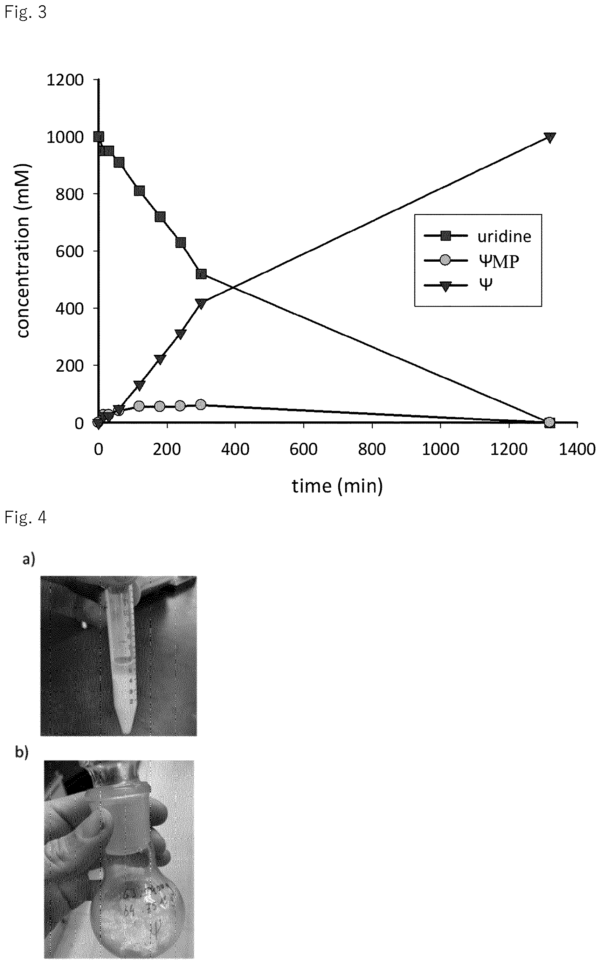

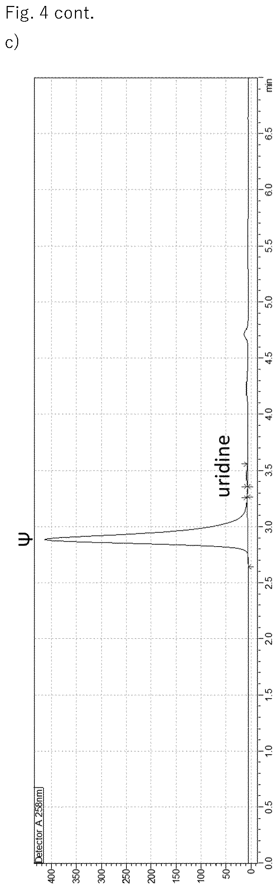

Synthesis was performed in 5 mL scale and 100 mM KH₂PO₄, 1 M uridine,1 mM MnCl₂, were dissolved in water and the pH was set to 7.0 with KOH. The reaction was initiated by addition of 0.25 mg/mL Ec_UP, 2.5 mg/mL EC_DeoB, 1.5 mg/mL Ec_YeiN and 0.2 mg/mL Ec_Yjjg. It was incubated at 30° C for 22 h. Ψ spontaneously crystallized from the reaction mixture and 1.2 g could be isolated by simple centrifugation (> 95% yield). The time course of Ψ synthesis is shown in Fig. 3. The produced Ψ is shown in Fig. 4 a) and Fig. 4 b). Ψ after lyophilization is shown in Fig. 4 b). HPLC analysis of isolated Ψ is shown in Fig. 4 c). HPLC and NMR analysis confirm the identity of the product and reported values agree well with literature [1].
The annotated NMR results of the product are given in the following:
¹H NMR (300 MHz, Deuterium Oxide ) of Ψ: δ 7.58 (s, H-6, 1H), 4.59 (d, J= 5.3 Hz, H-1', 1H), 4.21 (t, *J* = 5.4 Hz, H-2', 1H), 4.06 (t, *J* = 5.3 Hz, H-3', 1H), 3.97 - 3.90 (m, H-4', 1H,), 3.85 - 3.58 (m, H-5', 2H).
¹³C NMR (76 MHz, Deuterium Oxide) of Ψ: *δ* 165.31 (C-2), 153.58 (C-4), 141.54 (C-6), 110.51 (C-5), 83.37 (C-4'), 79.15 (C-1'), 73.36 (C-2'), 70.83 (C-3'), 61.52 (C-5').

### Example 3 - Enzymatic phosphorylation of Ψ MP

### SCHEME 3: Synthesis of pseudouridine-triphosphate (ΨTP).

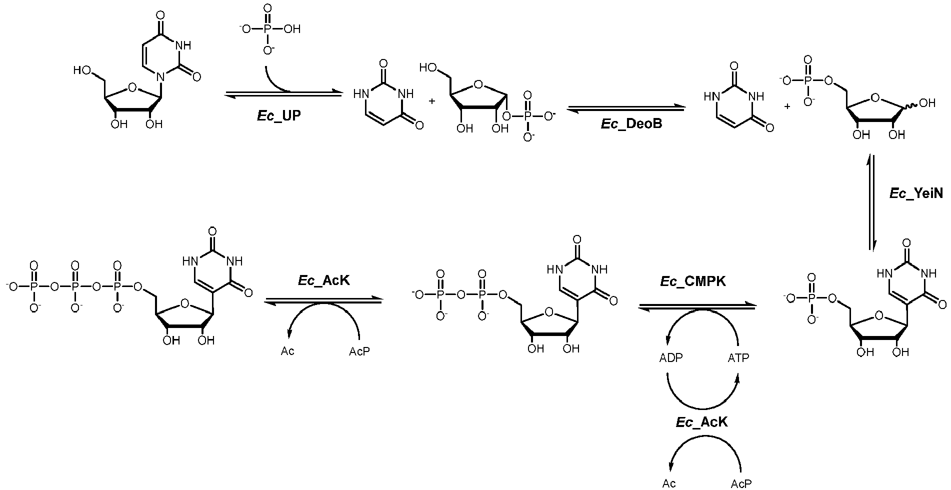

### Synthesis of ΨTP:

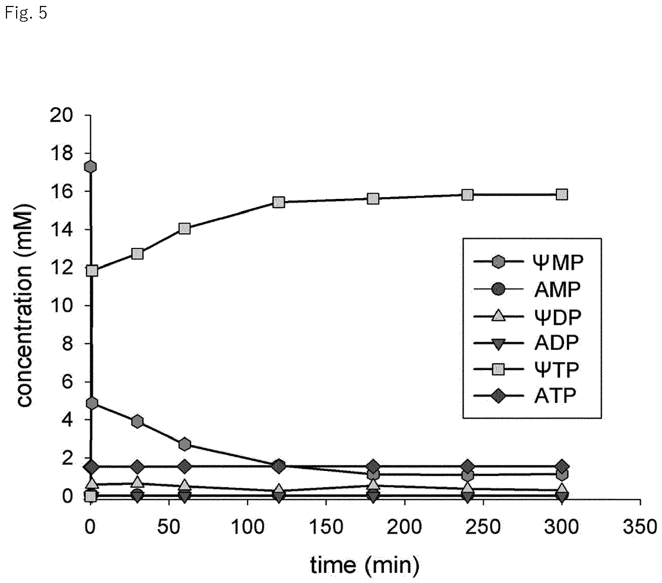

In a total volume of 400 µL, 17 mM Ψ MP (from the reaction mixture of the Ψ MP synthesis, not isolated), 1.25 mM ATP, 100 mM acetylphosphate (AcP), 5 mM MgCl₂, 50 mM HEPES and 50 mM TAPS pH 9.0 were mixed and the pH was adjusted to 9.0 using NaOH. The reaction was started by addition of 2 mg/mL Ec_CMPK and Ec_AcK. After 300 min of incubation at 30 ° C the reaction was completed with 95% conversion. Enzymes were heat inactivated (99 ° C for 7min) and removed by centrifugation. The time course of Ψ TP synthesis is shown in Fig. 5.

### Isolation of Ψ TP

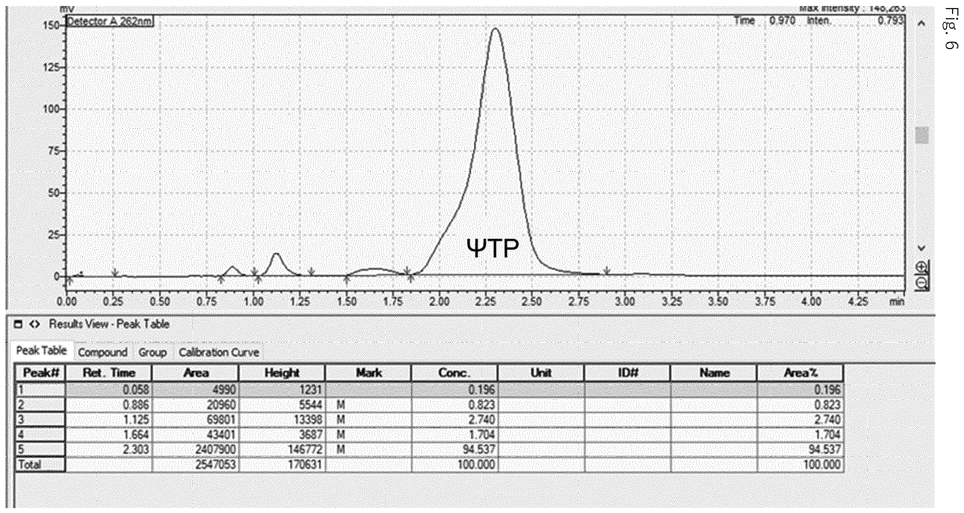

Cleared reaction mixture was loaded onto a 1 mL DEAE FF column and Ψ TP was eluted using a linear gradient of 500 mM ((NH₄)HCO₃) pH 7.5 in 20 CV. Fractions containing Ψ TP were pooled and lyophilized 2 times to evaporate the buffer. The final product was 95% pure based on HPLC measurements. The HPLC analysis of Ψ TP is shown in Fig. 6.

### References

[1] Pfeiffer, M., & Nidetzky, B. (2020). Reverse C-glycosidase reaction provides C-nucleotide building blocks of xenobiotic nucleic acids. Nature Communications, 11(1), [6270].
[2] Shanmugasundaram, M., Senthilvelan, A., Xiao, Z. & Kore, A.R. (2016) An Efficient Protection-Free One-Pot Chemical Synthesis of Modified Nucleoside-5' - Triphosphates, Nucleosides, Nucleotides and Nucleic Acids, 35:7, 356-362.
[3] Hildenbrand, J.C., Teleki, A. & Jendrossek, D. A universal polyphosphate kinase: PPK2c of Ralstonia eutropha accepts purine and pyrimidine nucleotides including uridine diphosphate. Appl Microbiol Biotechno/104, 6659-6667 (2020).
[4] Kulmer, S.T., Gutmann, A., Lemmerer, M., Nidetzky, B., (2017) Biocatalytic Cascade of Polyphosphate Kinase and Sucrose Synthase for Synthesis of Nucleotide - Activated Derivatives of Glucose, Advanced Synthesis and Catalysis, Volume 359, Issue 2, Pages 292-301, ISSN 1615-4150.
[5] Andries, O., Mc Cafferty, S., De Smedt, S. C., Weiss, R., Sanders, N. N., & Kitada, T. (2015). N(1)-methylpseudouridine-incorporated mRNA outperforms pseudouridine-incorporated mRNA by providing enhanced protein expression and reduced immunogenicity in mammalian cell lines and mice. Journal of controlled release : officialjournal of the Controlled Release Society, 217, 337-344.
[6] Chang YC, Herath J, Wang TH, Chow CS. Synthesis and solution conformation studies of 3-substituted uridine and pseudouridine derivatives. Bioorg Med Chem. 2008;16(5):2676-2686.
[7] Chaudhary, N., Weissman, D., & Whitehead, K. A. (2021). mRNA vaccines for infectious diseases: principles, delivery and clinical translation. Nature reviews. Drug discovery, 20(11), 817-838.
[8] Vita, A. & Magni, G. A one-step procedure for the purification of uridine phosphorylase from Escherichia coli. Anal. Biochem. 133, 153-156 (1983).
[9] Saheki, S., Takeda, A. & Shimazu, T. Assay of inorganic phosphate in the mild pH range, suitable for measurement of glycogen phosphorylase activity. Anal. Biochem. 148, 277-281 (1985).

## Claims

1. A method for the production of a C-nucleotide or C-nucleoside from the corresponding N-nucleoside, said method comprising the steps of:
a) providing an N-nucleoside and a phosphate donor;
b) adding an enzyme having phosphorylase activity, whereupon the N-nucleoside is converted into the corresponding nucleobase and sugar-phosphate moiety;
c) adding an enzyme having isomerase activity, whereupon the sugar-phosphate moiety is subjected to an intramolecular phosphoryl transfer; and
d) adding an enzyme having C-glycosidase activity, whereupon the nucleobase and the rearranged sugar-phosphate moiety yield the corresponding C-nucleoside-monophosphate.

2. The method of claim 1, wherein the method is performed as a one-pot reaction.

3. The method of claim 1 or 2, wherein the N-nucleoside is a pyrimidine nucleoside or a derivative thereof, preferably uridine, cytidine, or a derivative thereof.

4. The method of claim 3, wherein the uridine derivative is of general formula I, 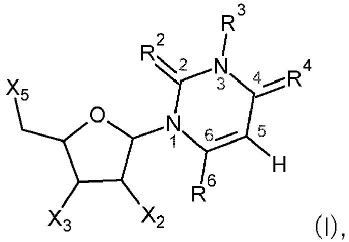 wherein
R² denotes O or S;
R³ denotes H, CH₃ or a group selected independently from one another among C₁₋₆alkyl, C₂₋₆alkenyl, C₂₋₆alkynyl, C₃₋₁₀cycloalkyl, C₄₋₁₆Cycloalkylalkyl, C₆₋₁₀aryl, 3-8 membered heterocycloalkyl, and 5-12 membered heteroaryl;
R⁴ denotes O or S;
R⁶denotes H, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl;
X₂ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl;
X₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl; and
X₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl.

5. The method of claim 3, wherein the cytidine derivative is of general formula II 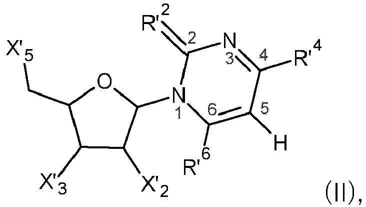 wherein
R'² denotes O or S;
R'⁴ denotes NH₂, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl;
R'⁶denotes H, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl;
X'₂denotes H, OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl;
X'₃ denotes H, OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl; and
X'₅ denotes OH, a halogen, or a group selected independently from one another among C₁₋₆alkyl, C₂₋₆alkenyl, C₂₋₆alkynyl, C₃₋₁₀Cycloalkyl, C₄₋₁₆cycloalkylalkyl, C₆₋₁₀aryl, 3-8 membered heterocycloalkyl, and 5-12 membered heteroaryl.

6. The method of any one of claims 1 to 5 wherein the phosphate donor is an inorganic phosphate, preferably phosphoric acid or a salt thereof, more preferably H₃P0₄, H₂PO₄⁻ , HPO₄²⁻ or PO₄³⁻ .

7. The method of any one of claims 1 to 6, wherein the enzyme having phosphorylase activity is a uridine phosphorylase or pyrimidine-nucleoside phosphorylase; more preferably EcUP, EcPNP, or a functionally active variant thereof.

8. The method of any one of claims 1 to 7, wherein the enzyme having isomerase activity is a phospho-mutase; preferably a phosphopentose-mutase; more preferably DeoB, or a functionally active variant thereof.

9. The method of any one of claims 1 to 8, wherein the enzyme having C-glycosidase activity is pseudouridine-monophosphate(Ψ MP)-glycosidase; preferably YeiN or a functionally active variant thereof.

10. The method of any one of claims 1 to 9, wherein said method comprises the following steps of:
a) providing uridine and a phosphate donor;
b) adding an enzyme having uridine phosphorylase activity, whereupon uridine is converted into uracil and ribose 1-phosphate;
c) adding an enzyme having phosphopentose-mutase activity, whereupon ribose 1-phosphate is converted to ribose 5-phosphate; and
d) adding an enzyme having Ψ MP-glycosidase activity, whereupon uracil and ribose 5-phosphate yield pseudouridine monophosphate (Ψ MP);
preferably said method is performed as a one-pot reaction.

11. The method of any one of claims 1 to 10, optionally further comprising the step of adding an enzyme having phosphatase activity, whereupon the phosphate group is removed from the C-nucleotide yielding the corresponding C-nucleoside, preferably pseudouridine (Ψ).

12. The method of claim 11, wherein the enzyme having phosphatase activity is calf intestine phosphatase (CIP), pyrimidine nucleoside monophosphate phosphatase (Yjjg), or a functionally active variant thereof.

13. The method of any one of claims 1 to 12, wherein the N1 atom of the nucleobase in the C-nucleotide or in the C-nucleoside is substituted by a suitable moiety.

14. Use of an enzyme mixture comprising an enzyme having phosphorylase activity, an enzyme having isomerase activity, an enzyme having C-glycosidase activity and optionally an enzyme having phosphatase activity for the production of a C-nucleotide or a C-nucleoside, preferably said enzyme having phosphatase activity is Yjjg.

15. A cell adapted to co-express the enzyme mixtures of claim 14.
